# EUROPEAN PATENT APPLICATION

(11) **EP 4 219 692 A1**
(43) Date of publication of application: **02.08.2023**
(21) Application number: 21871331.1
(22) Date of filing: 14.09.2021
(51) Int. Cl.: C12N 5/0793, C12Q 1/02, A61K 35/30

(54) **METHOD FOR DIFFERENTIATING PLURIPOTENT STEM CELLS INTO DOPAMINERGIC NERVE CELLS IN MIDBRAIN SUBSTANTIA NIGRA**

(30) Priority: 22.09.2020 CN 202011002775
(71) Applicant: Unixell Biotechnology, Shanghai 201207 (CN)
(72) Inventor: CHEN, Yuejun, Shanghai 200031 (CN); ZHOU, Wenhao, Shanghai 200031 (CN); XIONG, Man, Shanghai 200031 (CN)
(74) Representative: Grünecker Patent- und Rechtsanwälte PartG mbB
(86) International application number: PCT/CN2021/118183
(87) International publication number: WO 2022/062960

(57) **Abstract**

Provided is a specific method for differentiating pluripotent stem cells into dopaminergic (A9 mDA) nerve cells in the midbrain substantia nigra. Mature A9 mDA neurons are formed by means of differentiation, which can express the molecular markers of the midbrain substantia nigra dopaminergic neurons, comprising TH, FOXA2, EN1, LMX1A, NURR1 and GIRK2, but which rarely express the marker CALB of the ventral tegmental area dopaminergic neurons. The A9 mDA nerve cells are transplanted into the substantia nigra, and the axons thereof can specifically project to the target brain area which is innervated by endogenous substantia nigra dopaminergic neurons, i.e. the dorsal striatum; the transplanted A9 mDA neurons themselves exhibit the classic electrophysiological characteristics of the endogenous substantia nigra dopaminergic neurons, comprising a low-frequency spontaneous discharge frequency, and can induce sag by means of hyperpolarizing current stimulation; and transplanting the A9 mDA nerve cells into the substantia nigra or striatum of individuals with neurodegenerative diseases can alleviate motor deficits.

## Description

### Technical Field

The disclosure relates to the field of neural stem cells science. In particular, the disclosure relates to a method for differentiating pluripotent stem cells into dopaminergic nerve cells in midbrain substantia nigra.

### Background

Parkinson's disease (PD) is a long-term degenerative disease of central nervous system, with average age of onset is 60 years old and incidence rate is 1.7% in seniors over 65 years old. According to the international epidemiological survey, there were about 2 million PD patients in China in 2005, accounting for half of the global incidence. It is expected that by 2030, there will be nearly 5 million PD patients in China. Patients with Parkinson's disease often manifest movement disorders such as static tremor, hypertonia, bradykinesia, and postural instability. The pathological basis of Parkinson's disease is that the degeneration and loss of dopaminergic neurons in midbrain substantia nigra leads to a significant decrease of dopamine level in striatum, resulting in motor deficits.

Dopaminergic neurons were named for their ability to secrete the catecholamine neurotransmitter dopamine, with wide distribution in brain. Tyrosine hydroxylase (TH) is a marker of dopaminergic neurons, a rate-limiting enzyme for dopamine synthesis and is responsible for catalyzing the conversion of tyrosine to L-dopamine (L-DOPA). In midbrain, dopaminergic neurons are mainly distributed in three nuclei: the substantia nigra pars compacta (SNc), the ventral tegmental area (VTA) and the retrorubral field (RrF). The dopaminergic neurons of SNc are also called A9 mDA neurons, those of VTA are called A10 mDA neurons and those of RrF are A8 mDA neurons. The naming of A8-10 reflects their anatomical positioning. A9 mDA neurons and A10 mDA neurons are two important subtypes of midbrain dopaminergic neurons (mDA), and the two types of neurons are different in projection neuron circuits in addition to their locations. A9 mDA neurons project mainly to the dorsolateral striatum to form a nigrostriatal pathway in midbrain, which mainly regulates movements. A9 mDA neurons is a subgroup of dopaminergic neurons that is specifically lost in Parkinson's disease. A10 mDA neurons mainly project to the nucleus accumbens, cortex, olfactory cortex, amygdala, and so on, to form a mesolimbic cortex pathway, which is involved in regulating functions such as reward and emotion.

A9 mDA neurons and A10 mDA neurons exhibit different susceptibilities in Parkinson's disease. In the brain of Parkinson's patients, the A9 mDA neurons of the SNc degenerate preferentially, and the cells in the ventral side of the SNc are more sensitive than those in the dorsal side, while the A10 mDA neurons in the adjacent VTA are relatively spared. The intracellular calcium-binding protein calbindin (CALB) is used as a marker of A10 mDA neurons. Calbindin is used to selectively mark the group of dopaminergic neurons that are usually spared in the brains of PD patients and PD model animals, most of which are A10 mDA neurons. Another protein, G-protein-gated inward rectifier potassium channel (GIRK), regulates the activity of dopaminergic neurons by activating D2 or GABA receptors and generating a slow inhibitory postsynaptic potential. Among them, GIRK2 is specifically expressed in susceptible dopaminergic neurons (mostly A9 mDA neurons).

Clinically, there is no effective therapy to cure Parkinson's disease. Current treatments can only alleviate symptoms, but cannot prevent disease progression. Drug therapy is the most important treatment for Parkinson's disease by supplementing dopamine or enhancing the function of dopamine receptors to achieve therapeutic effects. Levodopa (L-DOPA) preparations are still the most effective drugs, but are only effective in the early stages of PD. With further loss of dopaminergic neurons, the drugs gradually lose effects and have significant side effects. Deep brain stimulation has also been used in the treatment of Parkinson's patients. However, same as drug treatments, surgical treatments can only alleviate symptoms, instead of curing the disease. Besides, because of the side effects of deep brain stimulation, this treatment is only suitable for part of patients. Transplanting dopaminergic neurons exogenously to replace the function of lost dopaminergic neurons in brain (stem cell therapy) is one of the promising therapeutic methods. Clinical experiments have found that by transplanting cells from the ventral midbrain (the brain region where the midbrain dopaminergic neural progenitors are located) of aborted fetuses to the striatum of patients, the motor deficits of some patients can be rescued for a long time, with no or only few drugs. Transplanted neural progenitors can differentiate into DA neurons and release DA. These clinical studies have demonstrated the great potential of stem cell therapy in PD treatment. However, the source of aborted fetal brain tissues is limited, with also ethical issues. Human pluripotent stem cells (hPSCs), including human embryonic stem cells (hESCs) and human induced pluripotent stem cells (hiPSCs), have the potential to differentiate into all types of cells in the body, and are the ideal cell sources for obtaining various functional cells for therapy. Following the principles of *in vivo* development, human pluripotent stem cells can be induced to differentiate into midbrain dopaminergic neurons. Transplanted into the striatum of PD model mice, these cells can survive and rescue the behavioral performance of Parkinson's disease, which brings new hope for the treatment of Parkinson's disease.

However, none of these studies clearly distinct subtypes of midbrain dopaminergic neurons (A9/A10) in transplanted cells. As mentioned above, different subtypes of endogenous dopaminergic neurons have completely different electrophysiological characteristics, innervated brain regions and physiological functions. Therefore, there is an urgent need to develop differentiation methods targeting different subtypes of midbrain dopaminergic neurons, especially for cell therapy of Parkinson's disease. It is necessary to develop an efficient differentiation method for enriching midbrain substantia nigra dopaminergic neurons. Besides, for differentiated dopaminergic neurons, it is necessary to verify their subtype specificity from multiple levels, including expressions of marker, characteristics of electrophysiology and characteristics of axonal projections.

### Summary

The purpose of the present disclosure is to provide a specific method for differentiating pluripotent stem cells into dopaminergic nerve cells in midbrain substantia nigra and cells or cell preparations obtained by the method.

In a first aspect of the present disclosure, there is provided a method for preparing midbrain substantia nigra dopaminergic neurons, comprising: (1) culturing stem cells in a medium containing neural induction agents, ie. supplementing components in consecutive multiple stages to accomplish induction; and (2) obtaining the stem cell-derived midbrain substantia nigra dopaminergic neurons from the culture.

In a preferred example, in (1), said multiple stages for induction with supplementary components are: first stage: adding SB431542, DMH-1, SHH and CHIR99021; second stage: adding SAG, SHH and CHIR99021; third stage: adding SHH, SAG and FGF8b; fourth stage: adding SHH and FGF8b.

In another preferred example, in the first stage: adding 1~15 µM (such as 2, 5, 8, 12, 14 µM) SB431542, 1~5 µM (such as 0.4, 0.6, 1, 3, 5, 10, 15, 18 µM) DMH-1, 200~1000 ng/mL (such as 300, 400, 600, 700, 800, 900 ng/mL) SHH, 0.1~1 µM (such as 0.2, 0.3, 0.4, 0.5, 0.6, 0.7, 0.8, 0.9 µM) CHIR99021.

In a more preferred example, in the first stage: adding 10±5 µM SB431542, 2±1 µM DMH-1, 500±200 ng/ml SHH, 0.4±0.2 µM CHIR99021. Further preferably, adding 10±2 µM SB431542, 2±0.5 µM DMH-1, 500±100 ng/ml SHH, 0.4±0.1 µM CHIR99021.

In another preferred example, in the second stage: adding 0.1~5 µM (such as 0.2, 0.5, 0.8, 1, 2, 3, 4 µM) SAG, 50~300 ng/ml (such as 80, 100, 150, 200, 250 ng/mL)SHH, 0.1~1 µM (such as 0.2, 0.3, 0.4, 0.5, 0.6, 0.7, 0.8, 0.9 µM) CHIR99021.

In a more preferred example, in the second stage: adding 2±1 µM SAG, 100±50 ng/ml SHH, 0.4±0.2 µM CHIR99021. Further preferably, adding 2±0.5 µM SAG, 100±20 ng/ml SHH, 0.4±0.1 µM CHIR99021.

In another preferred example, in the third stage: adding 5~100 ng/ml (such as 10, 15, 20, 30, 40, 50, 60, 70, 80 ng/mL; preferably 5~50 ng/ml) SHH, 0.1~5 µM (such as 0.2, 0.5, 0.8, 1, 2, 3, 4 µM) SAG, 5~200 ng/ml (such as 10, 15, 20, 40, 60, 80, 100, 150, 200, 250 ng/mL) FGF8b.

In a more preferred example, in the third stage: adding 20±10 ng/ml SHH, 0.5±0.2 µM SAG, 100±50 ng/ml FGF8b. Further preferably, adding 20±5 ng/ml SHH, 0.5±0.1 µM SAG, 100±20 ng/ml FGF8b.

In another preferred example, in the fourth stage: adding 5~100 ng/ml (such as 10, 15, 20, 30, 40, 50, 60, 70, 80 ng/mL) SHH, 5~80 ng/ml (such as 10, 15, 20, 30, 40, 50, 60, 70 ng/mL) FGF8b.

In a more preferred example, in the fourth stage: adding 20±10 ng/ml SHH, 20±10 ng/ml FGF8b. Further preferably, adding 20±3 ng/ml SHH, 20±3 ng/ml FGF8b.

In another preferred example, in said multiple stages: first stage: culturing for 6~8 days from the beginning; preferably 7±0.5 days; second stage: culturing for 6~8 days to 11∼13 days; preferably 12±0.5 days; third stage: culturing for 11~13 days to 18~20 days; preferably 19±0.5 days; fourth stage: culturing for 18~20 days to 31~33 days; preferably 32±0.5 days.

In another preferred example, wherein the stem cells comprise: embryonic stem cells or induced pluripotent stem cells; preferably, the stem cells are human stem cells, the embryonic stem cells or induced pluripotent stem cells are human embryonic stem cells or human induced pluripotent stem cells.

In another aspect of the present disclosure, there is provided a midbrain substantia nigra dopaminergic nerve cell, wherein it is prepared by any one of the method described above.

In another aspect of the present disclosure, there is provided a midbrain substantia nigra dopaminergic nerve cell, wherein it expresses molecular markers of midbrain substantia nigra dopaminergic neurons after differentiation for 5~10 days (such as 6, 7, 8, 9 days), said molecular markers comprising tyrosine hydroxylase (TH), FOXA2, EN1, LMX1A, NURR1 and/or GIRK2; while it rarely expresses the marker CALB (CB) of the ventral tegmental area dopaminergic neurons.

In another preferred example, the differentiation is *in vitro* differentiation, comprising: attachment and maturation *in vitro* and differentiation into mature midbrain substantia nigra dopaminergic neurons.

In another preference, the differentiation is *in vivo* differentiation, comprising: maturation *in vivo.*

In another preferred example, the rarely expressed marker CALB of the ventral tegmental area dopaminergic neurons comprises: expressing CALB less than 20%, preferably less than 15%, more preferably less than 12%, 10%, 8%.

In another preferred example, after the midbrain substantia nigra dopaminergic nerve cell being transplanted into the substantia nigra and differentiation, A9 mDA neurons are obtained and the axons thereof can specifically project to the target brain area-dorsal striatum which is innervated by endogenous substantia nigra dopaminergic neurons.

In another preferred example, the transplanted A9 mDA neurons themselves exhibit the classic electrophysiological characteristics of the endogenous substantia nigra dopaminergic neurons, comprising a low-frequency spontaneous discharge frequency, and can induce sag by means of hyperpolarizing current stimulation.

In another preferred example, the transplanted A9 mDA neurons in the substantia nigra or striatum of brains can alleviate motor deficits.

In another preferred example, the midbrain substantia nigra dopaminergic nerve cell is A9 mDA nerve cell; preferably, more than 80% of the cells express A9 mDA marker GIRK2 after differentiation for 5~10 days (such as 6, 7, 8, 9 days); more preferably, more than 85% of the cells express A9 mDA marker GIRK2.

In another preferred example, after differentiation for 5~10 days, more than 40% (such as 45%, 50%, 55%, 60%, 70%) of total cells or more than 50% (such as 55%, 60%, 65%, 70%, 75%, 80%) of the cells in TUJ1+ neurons exhibit the characteristics; more preferably, more than 50% (such as 55%, 60%, 65%, 70%, 75%) of total cells or more than 60% (such as 65%, 70%, 75%) of the cells in TUJ1+ neurons exhibit the characteristics.

In another aspect of the present disclosure, there is provided a midbrain substantia nigra dopaminergic neuron obtained from the differentiation of the midbrain substantia nigra dopaminergic nerve cell mentioned above; preferably, it expresses molecular markers of midbrain substantia nigra dopaminergic neurons, comprising TH, FOXA2, EN1, LMX1A, NURR1 and/or GIRK2, while it rarely expresses the marker CALB (CB) of the ventral tegmental area dopaminergic neurons.

In another aspect of the present disclosure, there is provided a use of any one of midbrain substantia nigra dopaminergic nerve cell mentioned above for preparing preparations (comprising cell cultures or isolates) in treating neurodegenerative diseases.

In another aspect of the present disclosure, there is provided a preparation for treating neurodegenerative diseases, wherein, it comprises: any one of midbrain substantia nigra dopaminergic nerve cell mentioned above; and pharmaceutically acceptable carriers.

In another preferred example, the preparation is also used as a graft (drug) for transplantation into the substantia nigra or striatum of the brain.

In another preferred example, the preparation is also used for preventing or treating motor deficits.

In another preferred example, the neurodegenerative diseases comprise (but not limited to): Parkinson's disease, Alzheimer's disease, Lewy body dementia, Huntington's disease, amyotrophic lateral sclerosis, nerve damage.

In another aspect of the present disclosure, there is provided a method for screening substances (including potential substances) for improving neurodegenerative diseases, wherein the method comprises: (1) treating a model system by a candidate substance, wherein the model system is neural circuit damaged or neural function damaged which comprises midbrain dopaminergic neurons (cells); and (2) evaluating, if the candidate substance can statistically promote (significantly promote, such as promote more than 10%, 20%, 50%, 80% and so on) dopaminergic neurons to repair damaged neural circuits in the brain or promote their remodeling of neural functions, then the candidate substance is a useful substance for repairing damaged neural circuits or reconstructing neural functions.

In another preferred example, the model system in step (1) is an animal model system, a tissue model system, an organ model system, a cell (cell culture) model system.

In another preferred example, step (2) comprises: observing the influence of the candidate substance to midbrain dopaminergic neurons, if it promotes (significantly promotes, such as promotes more than 10%, 20%, 50%, 80% and so on) midbrain dopaminergic neurons to repair nigra-striatal pathway, then it is a useful substance for repairing damaged neural circuits or reconstructing neural functions.

In another preferred example, step (2) comprises: observing the influence of the candidate substance to midbrain dopaminergic neurons, if it promotes (significantly promotes, such as promotes more than 10%, 20%, 50%, 80% and so on) pre- and post-synaptic integration of midbrain dopaminergic neurons, then it is a useful substance for repairing damaged neural circuits or reconstructing neural functions.

In another preferred example, step (2) comprises: observing the influence of the candidate substance to midbrain dopaminergic neurons, if it promotes (significantly promotes, such as promotes more than 10%, 20%, 50%, 80% and so on) the projection of axons of midbrain dopaminergic neurons to the dorsal region (Caudate putamen, CPu) of striatum, then it is a useful substance for repairing damaged neural circuits or reconstructing neural functions.

In another preferred example, step (2) comprises: observing the influence of the candidate substance to midbrain dopaminergic neurons, if it promotes (significantly promotes, such as promotes more than 10%, 20%, 50%, 80% and so on) the neural fiber formation of midbrain dopaminergic neurons, along with the endogenous nigra-striatal neural connected pathway, specific growth and extension to its endogenous target area-striatum to form neural connections with striatal neurons, and projection to the striatum, then it is a useful substance for repairing damaged neural circuits or reconstructing neural functions.

In another preferred example, step (2) comprises: the model system is an animal system, the animal has motor deficits, wherein the method also comprises: evaluating the motor abilities of animals, if the candidate substance can alleviate (significantly improve, such as improve more than 10%, 20%, 50%, 80% and so on) motor deficits, then it is a useful substance for repairing damaged neural circuits or reconstructing neural functions.

In another preferred example, step (1) comprises: treating a model system by a candidate substance; and/ or step (2) comprises: detecting the functions of dopaminergic neurons in the model system to damaged neural circuits in the brain, or detecting the functions of dopaminergic neurons to the remodeling of neural functions, or detecting the functions of dopaminergic neurons to the repairing of nigra-striatal pathway, or detecting the functions of dopaminergic neurons to pre- and post-synaptic integration, or observing motor deficits of animals; and compared with the control group, wherein the control group is the expressive system without adding the candidate substance; if the candidate substance can statistically promote dopaminergic neurons to damaged neural circuits in the brain or promote their remodeling of neural functions, or promote midbrain dopaminergic neurons to repair nigra-striatal pathway, or promote pre- and post-synaptic integration of midbrain dopaminergic neurons, or alleviate motor deficits of animals, then the candidate substance is a useful substance for repairing damaged neural circuits or reconstructing neural functions.

In another preferred example, the screening methods do not comprise methods directly aimed at treating diseases.

In another preferred embodiment, the candidate substances comprise (but not limited to): compounds, interacting molecules, biomacromolecules and so on.

In another preferred example, the method further comprises: performing further cell experiments, animal (such as mice, non-human primate) experiments, or clinical experiments in human of the obtained substances or potential substances, so as to further select and determine the substance useful for repairing damaged neural circuits or reconstructing neural functions from the candidate substances.

Other aspects of the present disclosure will be apparent to those skilled in the art based on the disclosure herein.

### Brief Description of Figures

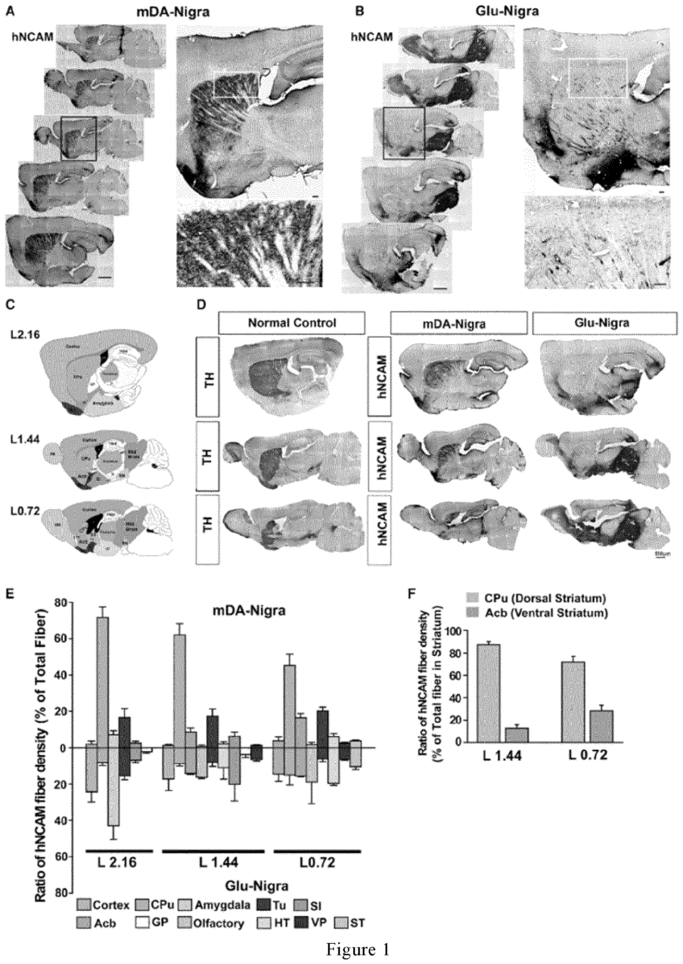

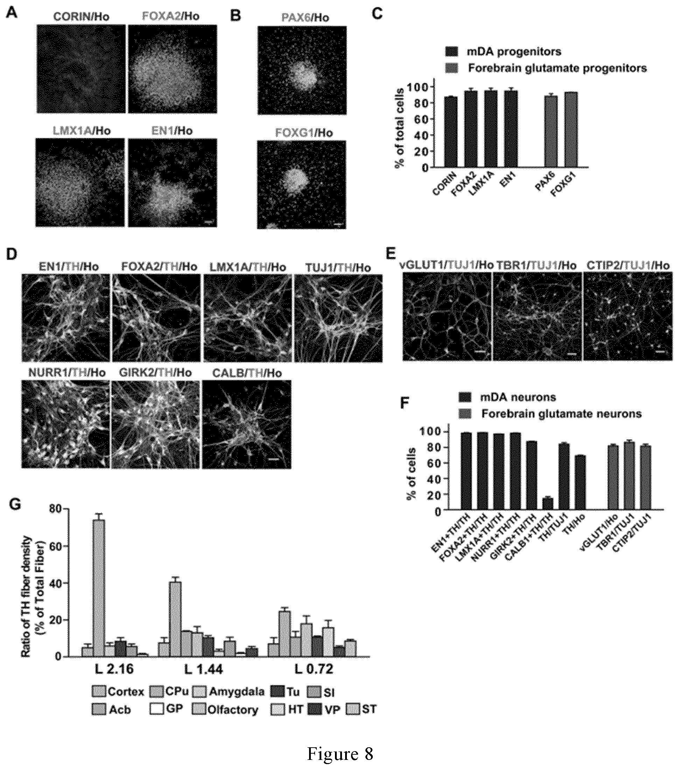

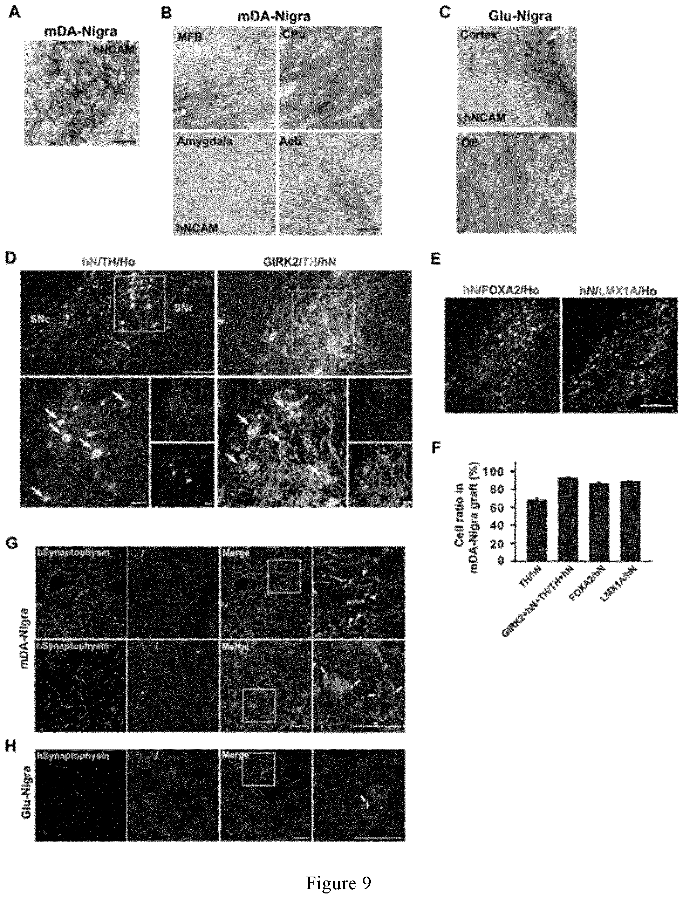

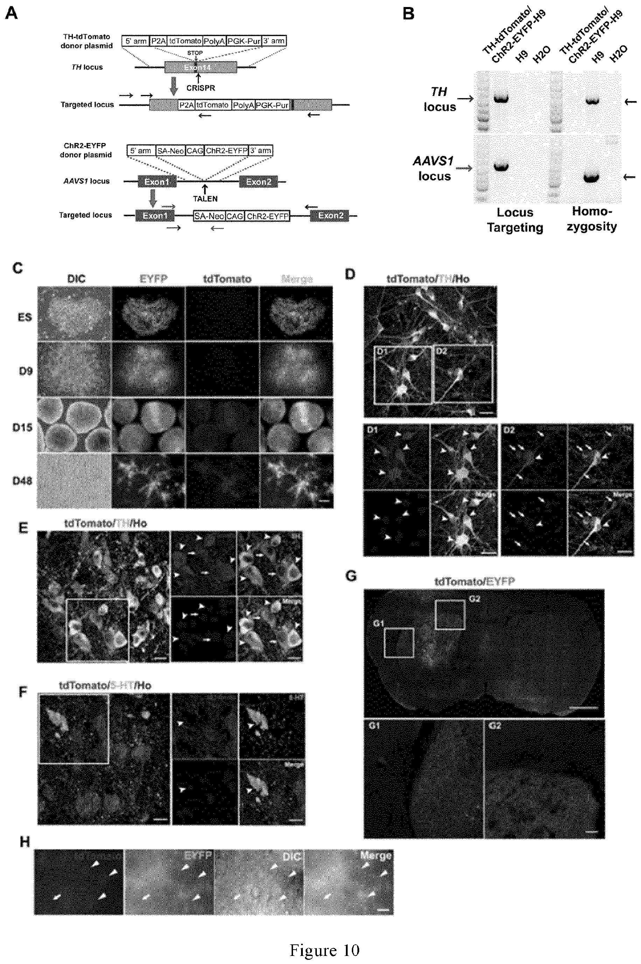

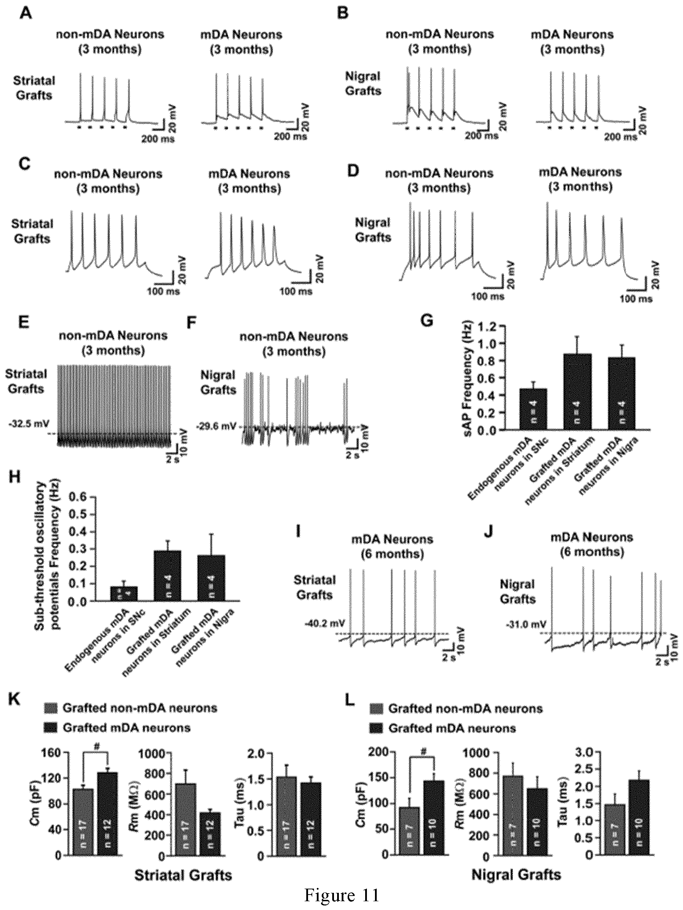

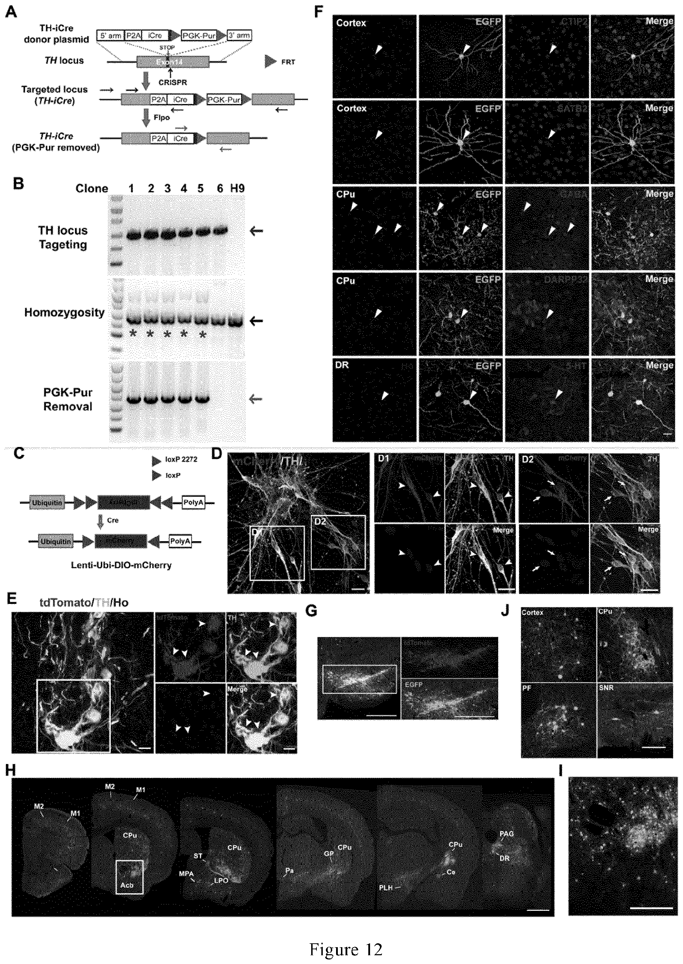

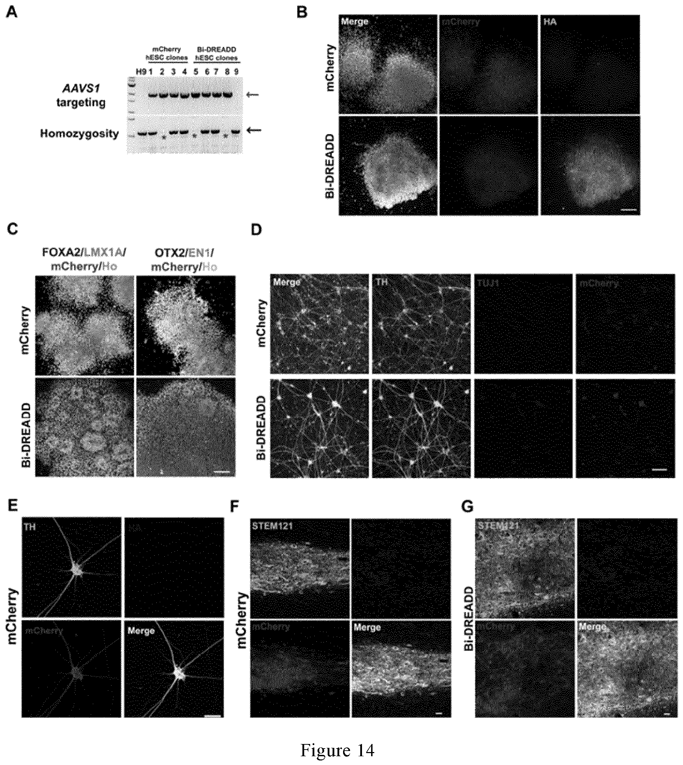

**Figure 1****. Axonal projections of nigrally transplanted human neurons.**
   (A and B) Immunohistochemistry of sagittal sections for hNCAM from a nigral graft with mDA (A) or Glu neurons (B). Scale bar, 250 µm. The black-boxed areas are amplified at the top right. The white-boxed areas are amplified at the bottom right. Scale bars, 25 µm for the amplified images.
   (C) Schematic sagittal diagram of anatomical structures in three representative planes.
   (D) Immunostaining for TH in wild-type mice (left panel) or in PD mice transplanted with mDA neurons (center panel) or Glu neurons (right panel) at corresponding sagittal planes.
   (E) Quantification of the regional distribution of hNCAM+ fibers in different areas. n = 8 for the mDA neuron group, n= 6 for the Glu neuron group.
   (F) Relative distribution of hNCAM+ fibers in the dorsal (CPu) and ventral striatum (Acb) from the two representative planes of mDA-transplanted mice. Images in (A), (B), and (D) are automatically stitched from multiple high-magnification images. See also Figure 8.

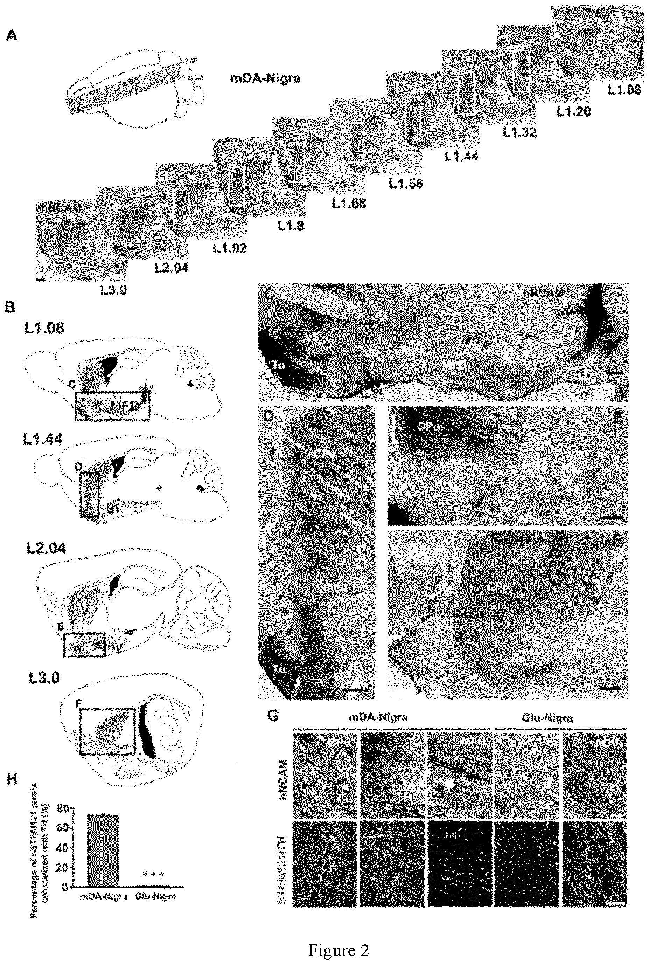

**Figure 2****. Axonal Projection Pathways of Nigrally Transplanted Neurons.**
   (A) A schematic of the approximate medial-lateral planes and the corresponding serial sagittal sections immunostained for hNCAM from the mouse brain transplanted with mDA neurons.
   (B) Neurolucida drawing of hNCAM+ axonal projection at different planes of sagittal sections. The boxed areas are magnified in (C)-(F).
   (C-F) High magnification illustrates the axonal projection pathways and territory. Scale bar, 250 µm. The red arrowheads in (C) indicate the hNCAM+ axons in the MFB. The red arrowheads in (D) indicate ascending hNCAM+ axons. Shown in (F) is the distribution of hNCAM+ fibers in the lateral striatum. The blue arrowheads in (D) and (F) indicate hNCAM+ fibers in the cortex.
   (G) The morphology of hNCAM+ fibers and co-labeling of human STEM121 and TH in fibers derived from mDA or Glu neuron grafts in different host brain regions. Scale bars, 50 mm (top panel) and 25 mm (bottom panel).
   (H) Quantification of the percentage of human STEM121 pixels colocalized with TH in the CPu of mice transplanted with mDA or Glu neurons. Data are represented as mean ± SEM. Student's t test. * * * p < 0.001.
   Images in (A) and (C-F) are automatically stitched from multiple high-magnification images. See also Figure 9.

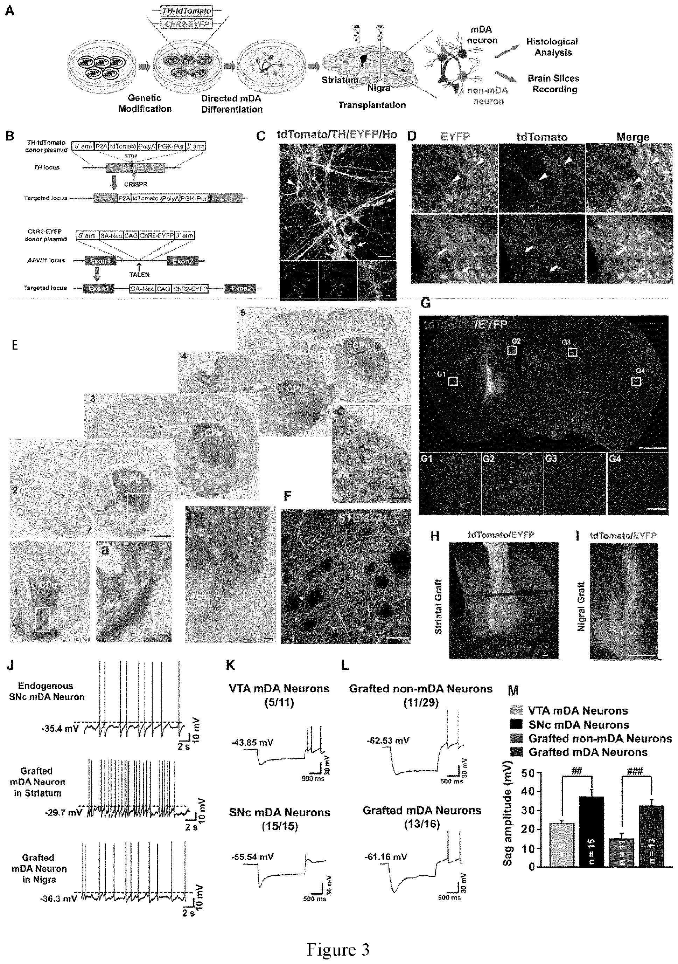

**Figure 3****. Axonal Projections and Electrophysiological Properties of Genetically Labeled Human mDA Neurons.**
   (A) The strategy for visualization and electrophysiological recording of grafted human mDA or non-mDA neurons.
   (B) The strategy for generation of the TH-tdTomato/ChR2-EYFP dual-locus knockin hESC line (TH-tdTomato/AAVS1-ChR2-EYFP hESCs).
   (C) Immunostaining of day 42 cultures derived from the above hESCs shows co-expression of tdTomato and EYFP in TH neurons (white arrowheads) and expression of EYFP but not tdTomato in TH-neurons (white arrows). Scale bar, 20 µm.
   (D) Immunohistochemistry images show that the nigral graft with transgenic human mDA neurons contains tdTomato+/EYFP+ mDA neurons (white arrowheads) and tdTomato-/EYFP+ non-mDA neuronal cells (white arrows).
   (E) Serial coronal sections immunostained for tdTomato from the PD mouse brain with a nigral graft. Scale bar, 1 mm. As shown in the Figure, boxed areas are magnified as indicated. Scale bar, 0.1mm. Dotted red arrows indicate ascending axons from the rostral-ventral part of the striatum.
   (F) Co-labeling of human STEM121 and tdTomato. Scale bar, 50 µm.
   (G - I) Coronal sections of the graft site, immunostained for tdTomato and EYFP from the PD mouse brain with a striatal (G and H) or nigral (I) graft. Boxed areas in(G) are magnified below. The image in (I) is a composite of two separate images for the top and bottom parts of the same graft. Scale bars, 1 mm, top panel in (G); 100 mm, bottom panel in (G); 250 µm in (H) and (I).
   (J) Typical traces of spontaneous action potentials (sAPs) in endogenous SNc mDA neurons from mDA neuron reporter mice (DAT - ere /Ai9) or striatally or nigrally grafted human mDA neurons 3 months after transplantation.
   (K-M) Typical traces of voltage sag measurements from endogenous medial VTA or SNc mDA neurons in mDA neuron reporter mice (K) or striatally grafted human non-mDA neurons or mDA neurons 6 months after transplantation (L). The numbers in parentheses represent the numbers of neurons displaying sag components among recorded cells. The sag amplitude is plotted in (M). The sample number for statistics is indicated in the column. Data are represented as mean ± SEM. Student's t test, ##p < 0.01, ##p < 0.001.
   Images in (E), (G), and (H) are automatically stitched from multiple high-magnification images. The image in (I) is stitched from two separate images for the upper part and lower part of the same section. See also Figure 10 and 11.

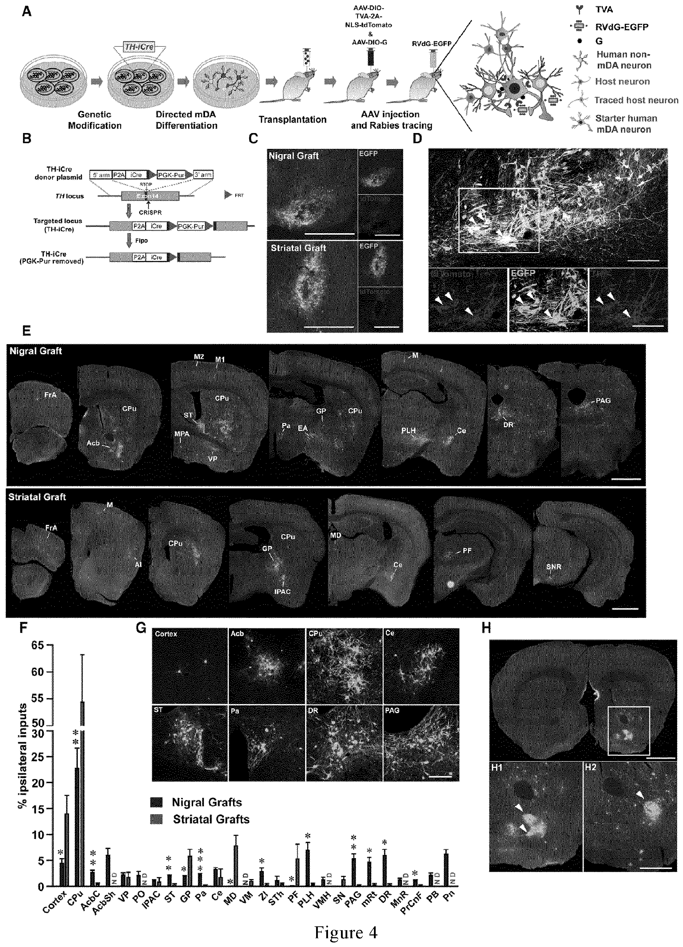

**Figure 4****. Rabies-Mediated Tracing of Inputs to Genetically Labeled Human mDA Neurons.**
   (A) The strategy for tracing inputs to genetically labeled human mDA neurons transplanted into the SN or striatum of PD mice.
   (B) Schematic diagram showing generation of the TH-iCre hESC line.
   (C) EGFP and tdTomato-expressing neurons in the graft site. Scale bar, 1 mm.
   (D) Immunohistochemistry images show expression of tdTomato, EGFP, and TH in neurons at the SN graft site. The white arrowheads indicate co-expression of EGFP and tdTomato in TH+ neurons. Scale bar, 100mm.
   (E) Serial coronal sections show distribution of traced host neurons (EGFP + / tdTomato-) to nigrally or striatally grafted human mDA neurons. Only the side ipsilateral to the graft is shown. Scale bar, 1 mm.
   (F) Quantification of ipsilaterally labeled inputs to nigrally or striatally grafted human mDA neurons, shown as a percentage of all ipsilateral inputs. Data are represented as mean ± SEM. Only brain regions with an average input percentage of more than 1% to nigral or striatal grafts are shown. n = 3 for striatal grafts, n=5 for nigral grafts. ND, not detected. Student's t test, ^{∗}p<0.05, ^{∗∗}p<0.01, ^{∗∗∗}p<0.001.
   (G) Magnified images of labeled input neurons to nigrally grafted mDA neurons in different host brain regions. Scale bar, 200mm.
   (H) Coronal section showing labeled input neurons to nigrally grafted mDA neurons in the Acb. The boxed area is magnified below (H1). An example distribution of input neurons from another animal is shown (H2). White arrowheads indicate a patch-like distribution of labeled input neurons. Scale bars, 1 mm (top image) and 0.5 mm (bottom images).
   Images in (C-E) and (H) are automatically stitched from multiple high-magnification images. Images in (G) are magnified from tiled images. See also Figure 12.

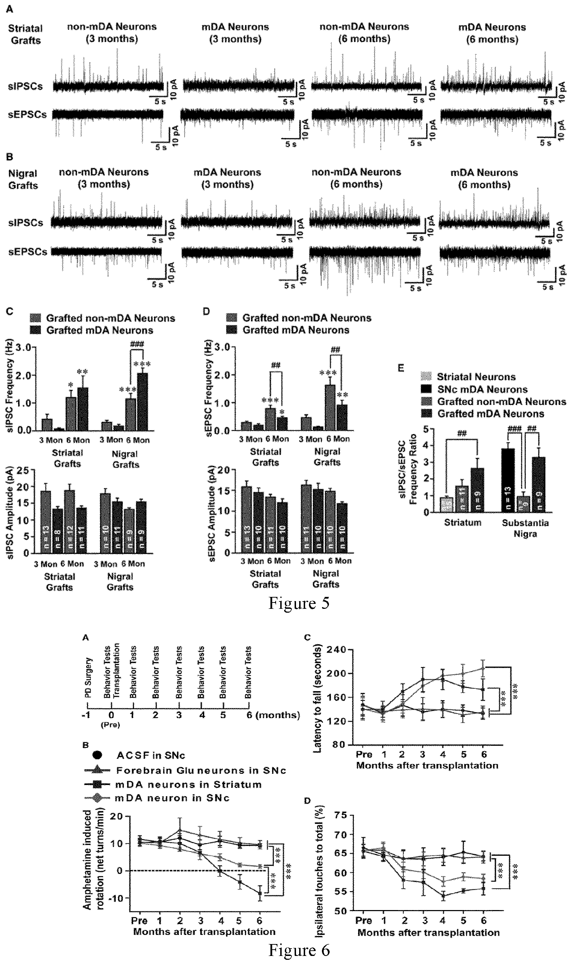

**Figure 5****. Electrophysiological Properties of Inputs to Human mDA or Non-mDA Neurons.**
   (A and B) Typical traces of sEPSCs and sIPSCs in striatally (A) or nigrally (B) grafted human mDA or non-mDA neurons 3 or 6 months after transplantation.
   (C and D) The frequency and amplitude of sIPSCs (C) and sEPSCs (D) were plotted. Data are represented as mean ± SEM. One-way ANOVA followed by Holm-Sidak post hoc test. ^{∗} p <0.05, ^{∗∗} p <0.01, *** p <0.001; comparison of 3-month and 6-month grafted mDA or non-DA neurons. ## p <0.01, ### p <0.001; comparison between grafted mDA and non-mDA neurons.
   (E) The sIPSC / sEPSC ratio in endogenous striatal or SNc neurons in wild-type SCID mice or non-mDA or mDA neurons in the striatum or nigra 6 months after transplantation was plotted. Data are represented as mean ± SEM. One-way ANOVA followed by Holm-Sidak post hoc test. ## p <0.01, ### p <0.001. The sample number for statistics is indicated in the column.
**Figure 6****. Behavioral Consequence of Transplanted Animals.**
   (A) The experimental process of the establishment of the animal model, transplantation, and behavioral analysis. These animals were tested monthly usingl behavior tests, including amphetamine-induced rotation, rotarod test, and cylinder test.
   (B) Amphetamine-induced rotation behavior changes over 6 months post-transplantation.
   (C) The rotarod test shows the changes in latency to fall before and after transplantation.
   (D) The cylinder test shows the preferential ipsilateral touches before and after transplantation.
      In all three behavioral tests, n = 11 for the nigral mDA group, n=8 for the nigral Glu group, n = 8 for the nigral ACSF group, and n = 8 for the striatal mDA group. Data are represented as mean ± SEM. One-way ANOVA followed by Holm-Sidak post hoc test. *** p < 0.001.

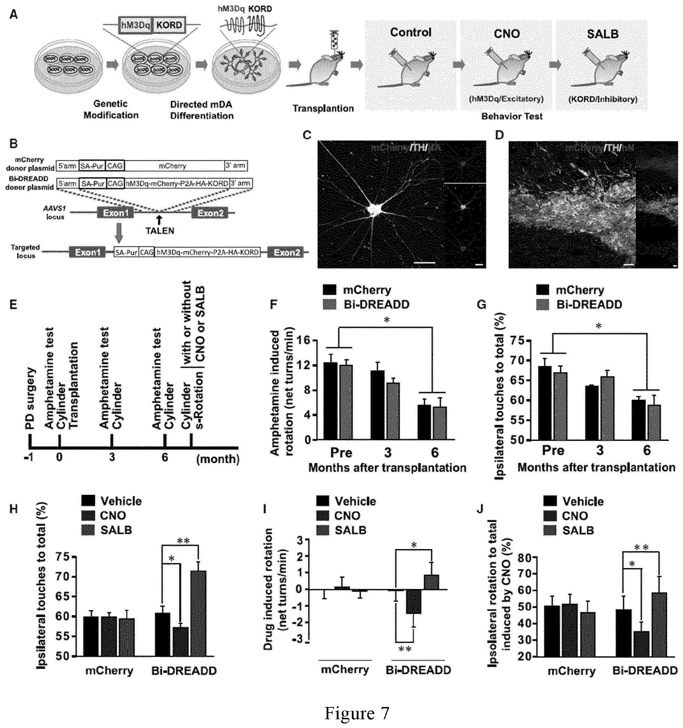

**Figure 7****. Bi-directional Control of PD Mice that Received a Transplant.**
   (A) The strategy for bi-directional regulation of human mDA neuron transplants in PD mice.
   (B) The strategy for generation of the mCherry and Bi-DREADD hESC lines.
   (C) Immunostaining shows co-expression of TH, hMsDq-mCherry, and hemagglutinin (HA)-tagged KORD in mDA neurons on day 42 of differentiation from Bi-DREADD hESCs. Scale bar, 50 mm.
   (D) Immunohistochemistry images show that nigrally grafted mDA neurons from Bi-DREADD hESCs co-expressed human nuclei (hNs), mCherry, and TH. Scale bar, 50 mm.
   (E) The experimental process of the animal model, transplantation, and behavioral analysis. S-rotation, spontaneous rotation.
   (F and G) Amphetamine-induced rotation and the cylinder test show the changes in rotation behavior (F) or preferential ipsilateral touches (G).
   (H) The cylinder test shows the changes in preferential ipsilateral touches of PD mice after treatment with vehicle, CNO, or SALB.
   (I and J) The spontaneous rotation test shows CNO-or SALB-induced changes in net ipsilateral rotations (I) and preferential ipsilateral rotations (J).
**Figure 8****. mDA and Glu neuron differentiation *in vitro* and TH fiber distribution *in vivo.***
   (A-B) Immunostaining of day-32 cultures derived from hESCs shows markers for mDA progenitors (A) and forebrain glutamate progenitors (B). Ho, Hoechst. Scale bar = 25µm.
   (C) Quantification of cellular differentiation presented in (A) and (B).
   (D-E) Immunostaining of day-42 cultures derived from hESCs shows markers for mDA neurons (D) and forebrain glutamate neurons (E). Scale bar = 25µm.
   (F) Quantification of cellular differentiation presented in (D) and (E).
   (G) Quantification of the regional distribution of TH+ fibers from wild type mice as in Figure 1D. n =5. TH+positive cell bodies in the cortex were included in our calculation.
**Figure 9****. Survival, axonal projection, and synaptogenesis of nigrally transplanted mDA and Glu neurons.**
   (A) Immunohistochemistry images for hNCAM from PD mouse brain with nigrally grafted mDA neurons show distribution and arborization of hNCAM+ fibers in CPu. The red arrows indicate bead-like structure of hNCAM+ fibers. Scale bar = 50 µm.
   (B) Immunohistochemistry images from lesioned mouse brain with nigrally grafted mDA neurons show morphology of hNCAM+ fibers in different brain regions. Scale bar = 125 µm.
   (C) Immunohistochemistry images for hNCAM from PD mouse brain with nigrally grafted Glu neurons show distribution and arborization of hNCAM+ fibers in cortex and OB (olfactory bulb). Scale bar = 125µm.
   (D) Immunohistochemistry images show that the grafted TH positive cells in nigra co-express human nuclei (hN) and GIRK2. Boxed regions are magnified below. Scale bar = 100 µm for large images, Scale bar = 25 µm for magnified images. Arrows indicate double positive cells.
   (E) Immunohistochemistry images show that the grafted human nuclei (hN) positive cells in nigra co-express FOXA2 and LMX1A. Scale bar = 100µm.
   (F) Quantification of cell identity presented in (D and E).
   (G) The mouse brain with nigrally grafted mDA neurons were stained for human-specific synaptophysin and TH (upper panel) or GABA (lower panel) in the host CPu. Boxed areas are magnified on the right. White arrowheads indicate co-localization of human-specific synaptophysin with TH along the TH fibers. White arrows indicate co-localization of human-specific synaptophysin with GABA around the GABA neuron cell body.
   (H) The mouse brain with nigrally grafted Glu neurons was stained forhuman-specific synaptophysin and GABA in the host CPu. Boxed areas are magnified on the right. White arrows indicate co-localization of human-specific synaptophysin with GABA around the GABA neuron cell body.
**Figure 10****. Establishment and characterization of TH-tdTomato/AAVS1-ChR2-EYFP hESC line.**
   (A) Schematic diagram of the genotyping strategy for TH-tdTomato/AAVS1-ChR2-EYFP hESC line. PCR primers for TH locus insertion or homozygosity are indicated by the red arrows and black arrows, respectively. PCR primers for AAVS1 locus insertion or homozygosity are indicated by the green arrows and blue arrows, respectively.
   (B) PCR genotyping of the TH-tdTomato/AAVS1-ChR2-EYFP hESC line. The expected PCR product for correctly targeted TH locus or AAVSI locus are ~1200 bp (red arrow) or -1000 bp (green arrow), respectively. Heterozygosity of the cell line is identified by the PCR product of -1000 bp (black arrow) or -650 bp (blue arrow) for TH locus or AAVSI locus, respectively. Those clones without -1000 bp or -650 bp PCR products are homozygous. The mother cell line H9 ESCs is included as control. TH-tdTomato/AAVS1-ChR2-EYFP hESC line is homozygous in both TH locus and AAVS1 locus;
   (C) DIC and fluorescent images show expression of EYFP and tdTomato in the ESCs or during mDA neuron differentiation of TH-tdTomato/AAVS1-ChR2-EYFP hESCs. tdTomato is expressed in the mid (D15) and terminal (D48) stages, but not in the ES or early stage (D9) stages of mDA neuron differentiation. Scale bar = 100µm.
   (D) Immunostaining of day 42 cultures derived from the above hESCs showsco-expression of tdTomato in TH+ neurons. Boxed regions are magnified below. White arrowheads indicate neurons with high expression of tdTomato and TH. White arrowheads indicate neurons with high expression of tdTomato and TH. Scale bar = 20µm.
   (E) Immunohistochemistry images show the expression of tdTomato in TH+ neurons in the nigral mDA graft. Boxed areas are magnified areas. White arrowheads and white arrows indicate neurons with high expression of tdTomato and TH or low expression of tdTomato and TH, respectively. Scale bar = 20µm.
   (F) Immunohistochemistry images show 5-HT positive serotonin neuron in the nigral mDA graft. Boxed areas are magnified areas. White arrowheads indicate tdTomato and 5-HT+ neurons.. Scale bar = 20µm;
   (G) Coronal section away from the graft site immunostained for tdTomato and EYFP from the PD mouse brain striatally grafted with mDA neurons derived from TH-tdTomato/AAVS1-ChR2-EYFP hESCs, showing specific projection of mDA neuron in the CPu, but not neighboring brain regions.
      Scale bar, 1 mm. Boxed regions are magnified below. Scale bar = 100µm. Images are automatically stitched from multiple high-magnification images.
   (H) DIC and fluorescent images of a slice from the PD mouse brain nigrally grafted with mDA neurons derived from TH-tdTomato/AAVS1-ChR2-EYFP hESCs. White arrowheads indicate tdTomato+ mDA neurons, and white arrow indicates tdTomato- non-mDA neurons in the EYFP+ graft. Scale bar = 50µm.
**Figure 11****. Electrophysiological examination of functional maturation of human mDA or non-mDA neurons grafted in nigra or striatum.**
   (A-D) Typical whole-cell path-clamp recording of blue light-induced action potentials (APs) (A and B) or current-induced APs (C and D) in striatally (A and C) or nigrally (B and D) grafted human mDA or non-mDA neurons at 3 months after transplantation.
   (E and F) Typical whole-cell path-clamp recording of spontaneous action potentials (sAPs) in striatally (E) or nigrally (F) grafted human non-mDA neurons at 3 months after transplantation. The dashed lines indicate threshold potentials.
   (G and H) The spontaneous action potential frequency (sAP) (G) and the sub-threshold oscillation potentials frequency (H) of endogenous SNc mDA neurons from mDA neuron reporter mice (DAT-Cre / Ai9), or striatally or nigrally grafted human mDA neurons at 3 months after transplantation were plotted. Data are represented as mean ± SEM. The sample number for statistics is indicated in the column. One-way ANOVA, p > 0.05.
   (I and J) Typical whole-cell path-clamp recording of spontaneous action potentials (sAPs) in striatally (I) or nigrally (J) grafted human mDA neurons at 6 months after transplantation. The dashed lines indicate threshold potentials.
   (K and L) The input resistance (Rm), membrane capacitance (Cm), and Tau of striatally (K) or nigrally (L) grafted human non-mDA and mDA neurons at 6 months after transplantation were plotted. Data are represented as mean ± SEM. The sample number for statistics is indicated in the column. Student-t test, # p<0.05.
**Figure 12****. Establishment of TH-icre hESC line and Rabies-mediated tracing of inputs to genetically labeled human and endogenous mDA neurons in mice.**
   (A) Schematic diagram of the genotyping strategy for TH-iCre hESC line. PCR primers for TH locus insertion or homozygosity are indicated by the red arrows and black arrows, respectively. PCR primer for PGK-Pur removal is indicated by the green arrows.
   (B) PCR genotyping of the TH-iCre hESC line. The expected PCR product for correctlytargeted TH locus are -1000 bp (red arrow). Homozygous clones are identified by the PCR product of~1000bp (black arrow). Those clones without PCR products are homozygous. The expected PCR product for PGK removal is~750 bp (green arrow). The mother cell line (H9 ESCs) is included as control. Heterozygous clones in TH locus with PGK-Pur removal (red asterisks) are selected for experiments.
   (C) Schematic depiction of lentivirus encoding Cre-dependent expression of mCherry driven by the ubiquitin promoter (Lenti-Ubi-DIO-mCherry).
   (D) mDA neuron cultures derived from TH-icre hESCs infected by Lenti-Ubi-DIO-mCherry show mCherry expression in the TH⁺ mDA neurons. Boxed areas are magnified right. White arrowheads indicate co-expression of mCherry and TH in mDA neurons. White arrows indicate mCherry expressing neurons with low TH expression. Scale bar, 20 µm.
   (E) Immunohistochemistry images show co-expression of tdTomato and TH in striatally grafted mDA neurons derived from TH-iCre hESC line infected by AAV-DIO-TVA-2A-NLS-tdTomato 6 months after transplantation. Boxed areas are magnified right. White arrowheads indicate co-expressed neurons. Scale bar, 20 µm.
   (F) Immunohistochemistry images show CTIP2+ or SATB2+ neurons in cortex, GABA+ or DARPP32+ neurons in CPu, 5-HT+ neuron in DR connected to nigrally grafted human mDA neurons. White arrowheads indicate co-expressed neurons. Scale bar, 100 µm.
   (G) Rabies-mediated trans-synaptic tracing of endogenous mDA neurons. Confocal images show EGFP and tdTomato expressing neurons in the SNc of DAT-Cre mice. Scale bar = 0.5 mm.
   (H) Series of coronal sections show distribution of traced neurons (EGFP+/tdTomato-) for endogenous mDA neurons in date - cre mice. Only the side ipsilateral to the graft is shown. Scale bar = 1 mm.
   (I) Magnification of the Boxed area. (I) Magnified image shows patch-like distribution of labeled input neurons to endogenous mDA neurons. Scale bar = 0.5 mm.
   (J) High-magnified images of labeled input neurons to striatally grafted mDA neurons in different host brain regions. Scale bar, 200 µm.
   (G-H) Images in (G) and (H) are automatically stitched from multiple high-magnification images.
   (I-J) Images in (I) and (J) are magnified from tiled images.

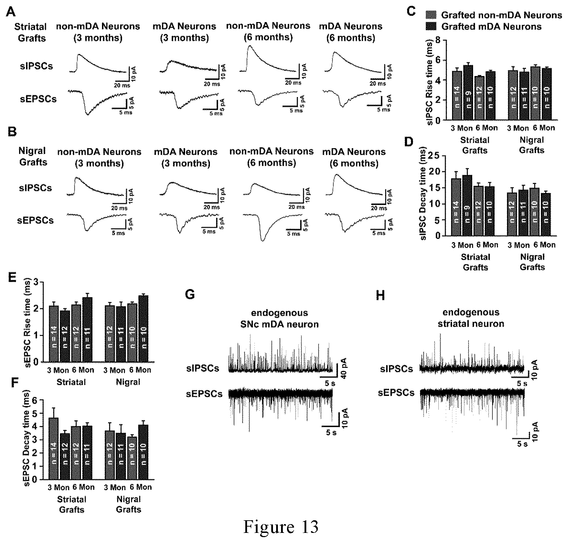

**Figure 13****. Functional inputs to endogenous neurons and the kinetics of sIPSCs and sEPSCs in grafted neurons.**
   (A and B) Representative traces from spontaneous excitatory postsynaptic currents (sEPSCs) and spontaneous inhibitory postsynaptic currents (sIPSCs) in striatal (A) or nigral (B) grafted non-mDA or mDA neurons in the brain slices at 3 or 6 months after transplantation.
   (C-F) Quantitative analysis of isolated sEPSCs and sIPSCs in A and B. Data are represented as mean ± SEM. The sample number for statistics is indicated in the column. One-way ANOVA followed by Holm-Sidak post hoc test. p> 0.05. (G and H) Typical whole-cell path-clamp recording of sEPSCs and sIPSCs in endogenous lateral SNc mDA.
   (G) or striatal neurons (H) of the brain slices from wild type SCID mice
**Figure 14****. Establishment and characterization of mCherry-and Bi-DREADD-expressing hESC lines.**
   (A) PCR genotyping of mCherry-or Bi-DREADD-expressing hESC clones. The expected PCR products for correctly targeted AAVSI locus are ~2000bp (red arrows). Homozygous clonesare identified by those without PCR products of ~650 bp (black arrow) in homozygosity test, and clones with ~650 bp PCR products are heterozygous. Homozygous clones (red asterisk) are selected for experiments.
   (B) Immunostaining shows expression of mCherry, hM3Dq-mcherry or HA-tagged KORD in mCherry or Bi-DREADD hESCs. Scale bar = 50µm.
   (C) Immunostaining for mDA neuron progenitor markers at day 16 of differentiation from mCherry or Bi-DREADD hESCs. Ho, Hoechst. Scale bar = 50µm.
   (D) Immunostaining of day 42 cultures derived from mCherry-or Bi-DREADD-expressing hESCs shows markers for mDA neurons. Scale bar = 50µm.
   (E) Immunostaining shows co-expression of TH, mCherry, but not HA-tagged KORD in mDA neurons at day 42 of differentiation from mCherry hESCs. Scale bar = 50µm.
   (F and G) Immunohistochemistry images show that the nigrally grafted mDA neurons derived from mCherry hESCs (F) or Bi-DREADD hESCs (G) co-expressed transgene mCherry or hM3Dq-mCherry and human STEM121. Scale bar = 20µm.

### Detailed Description

The inventors have deeply studied and revealed a specific method for differentiating pluripotent stem cells into midbrain substantia nigradopaminergic nerve cells. The differentiated mature A9 mDA neurons can express the molecular markers of the midbrain substantia nigra dopaminergic neurons, comprising TH, FOXA2, EN1, LMX1A, NURR1 and GIRK2, while rarely express the marker CALB of the ventral tegmental area dopaminergic neurons . The A9 mDA nerve cells can be transplanted into the substantia nigra, and the axons thereof can specifically project to the target brain area-dorsal striatum which is innervated by endogenous substantia nigra dopaminergic neurons; the grafted A9 mDA neurons receive more inhibitory but less excitatory inputs, whose regulation is similar to that of the endogenous substantia nigra dopaminergic neurons; the transplanted A9 mDA neurons themselves exhibit classic electrophysiological characteristics of the endogenous substantia nigra dopaminergic neurons, comprising a low-frequency spontaneous discharge frequency, and can induce sag by means of hyperpolarizing current stimulation; transplanting the A9 mDA nerve cells into the substantia nigra or striatum of individuals with neurodegenerative diseases can alleviate motor deficits.

### Terms

As used herein, the term "treatment" or "treat" here comprises preventive (e.g., prophylactic), curative, or palliative treatment of a mammal, especially human; and comprises (1) preventing, treating, or alleviating a disease (such as cancer) in an individual, wherein the individual is at high risk of developing the disease, or has the disease but has not yet been diagnosed; (2) inhibiting a disease (eg, inhibiting its occurrence); or (3) alleviating a disease (eg, alleviating symptoms associated with the disease).

As used herein, the "midbrain substantia nigra dopaminergic nerve cells", "stem cell-derived midbrain substantia nigra dopaminergic nerve cells" and "A9 mDA nerve cells" may be used! referenced interchangeably.

As used herein, the "cells" comprises "a population of cells" and also comprises "cell cultures".

As used herein, "individual", "patient", "participant" or "subject" refers to animals (such as rodents, primates) comprising humans, which can receive treatments of cells in the present disclosure (midbrain substantia nigra dopaminergic nerve cells) or cell preparations.

As used herein, "prevention" comprises "prevention", "alleviation" and "treatment".

As used herein, "neural circuit" refers to connected neurons with different properties and functions in brain through various forms; in the present disclosure, the nigra-striatal neural circuit is particularly concerned.

As used herein, "pharmaceutically accecptable" ingredients refers to substances suitable for use in humans and/or mammals without undue adverse side effects (such as toxicity), ie. with reasonable benefit/risk ratio. The term "pharmaceutically accecptable carrier" refers to a carrier for the administration of a therapeutic agent, comprising various excipients and diluents. The term refers to a pharmaceutical carrier that is, by itself, not essential active ingredients and is not unduly toxic after administration.

As used herein, "effective amount" refers to the amount of agents (cells or cell preparations in the present disclosure) that are sufficient to obtain desired curative effects. Effective amounts also comprise instances where the therapeutically beneficial effects of the agents outweigh toxic or detrimental effects thereof. Effective amount of agents does not necessarily cure diseases or disorders, but can delay, hinder or prevent the occurrence of the diseases or disorders, or alleviate the symptoms associated with the diseases or disorders. A therapeutically effective amount may be divided into one, two or more doses and administered once, two or more times in an appropriate dosage form within a given period.

### Stem cell-derived neurons and its preparation

The present disclosure provided midbrain substantia nigra dopaminergic nerve cells (or cell populations), wherein they are mainly A9 mDA nerve cells.

As a preferred example of the present disclosure, in stem cell-derived neurons obtained by the present disclosure, more than 80% express the A9 mDA neurons marker GIRK2; more preferably, more than 85% express the A9 mDA neurons marker GIRK2.

As a preferred example of the present disclosure, the midbrain substantia nigra dopaminergic neurons express markers of FOXA2, LMX1A and EN1. If further differentiated for about a week, they also express tyrosine hydroxylase (TH) and EN1, FOXA2, LMX1A and NURR1 (preferably more than 40% of total cells or more than 50% of cells in TUJ1+ neurons exhibit said characteristics; more preferably, more than 50% of the total cells or more than 60% of the cells in TUJ1+ neurons exhibit said characteristics).

The present disclosure also provided a method for preparing midbrain substantia nigra dopaminergic nerve cells *in vitro,* comprising: (1) culturing stem cells in a medium containing neural induction agents; and (2) obtaining the midbrain substantia nigra dopaminergic nerve cells from the culture.

As a preferred example of the present disclosure, in (1), adding neural induction agents into the medium; and inducing separately in multiple stages with supplementary components: first stage: adding SB431542, DMH-1, SHH and CHIR99021; second stage: adding SAG, SHH and CHIR99021; third stage: adding SHH, SAG and FGF8b; fourth stage: adding SHH and FGF8b.

As a preferred example of the present disclosure, the inventors also optimized the timing of addition of each supplementary component. Preferably, in multiple stages: first stage: culturing for 6~8 days from the beginning; preferably 7±0.5 days; second stage: culturing for 6~8 days to 11~13 days; preferably 12±0.5 days; third stage: culturing for 11~13 days to 18~20 days; preferably 19±0.5 days; fourth stage: culturing for 18~20 days to 31~33 days; preferably 32±0.5 days.

As a preferred example of the present disclosure, the inventors also optimized the amount of each supplementary component. The optimization of added amounts is beneficial for obtaining/enriching specific midbrain substantia nigra dopaminergic nerve cells.

Midbrain substantia nigra dopaminergic nerve cells obtained by optimized method in the present disclosure exhibit excellent effects, comprising: repairing damaged neural circuits in brain or reconstructing neural functions; more specifically comprising: forming neural fibers in substantia nigra, projecting to the striatum to repair; or forming synaptic connections between neurons and target cells in brain to repair; projecting axons to the caudate putamen (CPu) to repair; forming neural fibers, along with the endogenous nigra-striatal neural connected pathway, growing specifically and extending to its endogenous target area-striatum to form neural connections with striatal neurons, and projecting to the striatum to repair.

### Restorative effects of stem cell-derived nerve cells

Neurons are the basic functional unit of the brain. There are thousands of different types of neurons in brains of individuals. Complex and precise network connections (neural circuits) are formed between neurons, which is the basis for individuals to perceive the world, think and behave. Many neurological diseases, including stroke, cerebral trauma, and neurodegenerative diseases (Parkinson's disease and Alzheimer's disease, etc.), can lead to the loss of neurons and damage to neural connections in brain, resulting in severe neurological dysfunctions, such as hemiplegia, slow movements, muscle stiffness, impaired learning and memorable abilities, etc. However, the abilities of adult mammals, including humans, to regenerate nerves in brain are very limited. The loss of these neurons leads to the destruction of nerve connections and impaired nerve functions, with lack of effective treatments for diseases clinically. The key to stem cell therapy for neurological diseases is the restoration and functional reconstruction of damaged neural circuits. However, the precise network connections between neurons in brain of the individual are gradually formed during development, involving complex mechanisms of nerve fiber growth. In adult diseased brain environment, whether transplanted nerve cells can grow into nerve fibers, bridge "lost-connected" upstream and downstream brain regions, and repair damaged neural circuits still remains unclear. More importantly, is this restorative effect the result of random integration of transplanted cells or specific repair? What are the mechanisms and principles behind it? These are key issues to be solved urgently in the field of stem cell therapy for neurological diseases.

In view of the above problems, the inventors used Parkinson's disease as a disease model to study the feasibilities and mechanisms of transplanting stem cell-derived nerve cells into the adult brain to repair damaged neural circuits. Parkinson's disease is the world's second largest neurodegenerative disease mainly manifested by static tremor, myotonia, bradykinesia, and so on. Progressive loss of dopaminergic neurons in the brain substantia nigra is the main reason, resulting in the destruction of the neural connections between the substantia nigra and the striatum, then causes insufficient dopamine secretion in the striatum, and eventually leads to motor deficits of patients. The inventors are committed to human stem cell neural differentiation technology for different types of neurons, and based on this, an efficient method for human stem cells to differentiate into midbrain substantia nigra dopaminergic neurons has been established. Furthermore, human stem cells were genetically marked by gene editing technology, so that human dopaminergic neurons and their nerve fibers derived from stem cells could be specifically traced. The inventors transplanted genetically-marked human dopaminergic neurons into the damaged substantia nigra of Parkinson's disease model mice, and found that the human dopaminergic neurons transplanted in the brain substantia nigra grew a large number of nerve fibers along with the endogenous nigra-striatum neural connected pathway, grew specifically and extended to its endogenous target area-striatum to form neural connections with striatal neurons, and most of the nerve fibers projected to the striatum. By genetic technology and rabies virus-mediated tracing technology, the inventors further traced the upstream neural innervation received by transplanted human dopaminergic neurons and found that transplanted human dopaminergic neurons received a similar neural innervation to endogenous substantia nigra dopamine. Studies on the electrophysiological function of neurons have found that transplanted human dopaminergic neurons exhibit similar electrophysiological characteristics to endogenous dopaminergic neurons in the substantia nigra of animals, regulated by similar neurotransmitters. These results indicated that human dopaminergic neurons transplanted into the brains of Parkinson's disease model animals specifically repaired and reconstructed the damaged nigra-striatal neural connections, and their structures and functions were highly consistent with endogenous neural connections. Finally, by behavioral tests, the inventors found that the motor deficits of animals in the cell-transplanted group gradually improved with the prolongation of the transplantation. However, after inhibiting the activities of transplanted nerve cells by chemical genetics technology, the improvement of animal's motor functions disappeared, suggesting that reconstructed neural functional connectivity by transplanted cells mediated the recovery of behavior in model animals. Interestingly, the inventors transplanted another type of nerve cells, human cortical glutamatergic neurons, into the substantia nigra of Parkinson's disease model animals. The nerve fibers mainly projected to the cortex and olfactory bulb brain regions, with almost no projections in the striatum, which is hardly to repair damaged nigra-striatal neural circuit, also with motor deficits of model animals not be improved, indicating that only specific types of cells can repair specific neural functional circuits.

The present disclosure suggests that by transplanting nerve cells derived from stem cells, damaged nerve connections in the brain of adults can be repaired structurally and functionally and neural functions can be reconstructed. At the same time, the present disclosure also found that different types of nerve cells show different restorative effects on circuits, suggesting that for neurological diseases caused by the loss of different types of neurons, it is necessary to transplant specific nerve cells for circuit repair and treatment. These findings provide new ideas and theoretical basis for the treatment of brain injury and neurodegenerative diseases. Now the main types of nerve cells in the human brain can be efficiently obtained *in vitro* through stem cell neural differentiation technology. The development of stem cell technology will bring new hopes for the treatment of many neurological diseases.

Therefore, based on new findings of the inventors, technical solutions of the present disclosure have the following characteristics: (1) the dopaminergic neurons derived from stem cells (such as human embryonic stem cells) have the characteristics of midbrain substantia nigra dopaminergic neurons; (2) functional input depends on the type of transplanted neurons rather than the location of transplantation; (3) midbrain dopaminergic neurons can precisely restore the nigra-striatal pathway; (4) functionally repaired nigra-striatal pathway restored motor functions in animal models of neurodegenerative diseases such as Parkinson's disease.

### Drug screening

The inventors transplanted midbrain dopamine (mDA) or glutamate (Glu) cortical neurons derived from human embryonic stem cells (hESCs) into the substantia nigra or striatum of animal PD models, and found that the transplanted cells were widely integrated with the circuits in the host. Axonal pathways towards the dorsal striatum are determined by the type of transplanted neuron. Presynaptic inputs are largely dependent on the graft site, and inhibitory and excitatory inputs are determined by the type of transplanted neuron. hESC-derived mDA neurons exhibited characteristics of A9 neurons and restored the function of reconstituted nigra-striatal circuits to mediate improvements in motor functions. These results demonstrate similarities in cell-type-specific presynaptic and postsynaptic integrations between transplanted reconstructed circuits and endogenous neural networks, highlighting the abilities of hPSC-derived neuron subtypes in the adult brain for specific circuit repair and functional recovery.

Based on the new findings of the inventors, substances that repair damaged neural circuits in brain or remodel neural functions can be screened. Drugs useful for treating brain damage, neurodegenerative diseases, etc. can be found from the substances.

Therefore, the present disclosure provided a method for screening substances (including potential substances) for repairing damaged neural circuits or reconstructing neural functions, wherein the method comprises: (1) treating a model system by a candidate substance, wherein the model system is neural circuit damaged or neural function damaged, and it comprises dopaminergic neurons (dopaminergic nerve cells) in the midbrain; and (2) detecting the model system, if the candidate substance can statistically promote dopaminergic neurons to damaged neural circuits in the brain or promote their remodeling of neural functions, then the candidate substance is a useful substance for repairing damaged neural circuits or reconstructing neural functions.

Combined with research results of the present disclosure, after analyzing the effects of candidate substances on the repair of midbrain dopaminergic neurons for the nigra-striatal pathway, the presynaptic and post-synaptic integration of midbrain dopaminergic neurons, the ability of midbrain dopaminergic neuron axonal projections to dorsal regions and/or the athletic ability of animal models, the effectiveness of the candidate substances (potential substances or candidate drugs) can be determined.

Combined with research results of the present disclosure, by systematically analyzing the effects of candidate substances on the growth of midbrain dopaminergic neurons into nerve fibers, the specific growth and extension to its endogenous target area-striatumto form neural connections with striatal neurons and the projection to the striatum, the effectiveness of the candidate substances (potential substances or candidate drugs) can be determined.

In a preferred example of the disclosure, during screening, in order to more easily observe the changes before and after the treatment of candidate substance, a control group (Control) without adding the candidate substance (such as blank control or placebo control) can also be set.

On the other hand, the present disclosure also provided potential substances obtained by the screening method. These preliminary screening substances can constitute a library for screening, so that people can finally screen for substances that can be really useful for repairing damaged neural circuits in brain or remodeling neural functions.

### Pharmaceutical preparation

The present disclosure also provided a pharmaceutical composition (preparation), wherein it comprises effective amount (eg, 0.000001-50wt%; preferably 0.00001-20wt%; more preferably 0.0001-10wt%) of midbrain substantia nigra dopaminergic nerve cells prepared by the method of the present disclosure, and a pharmaceutically acceptable carrier.

The term "pharmaceutically accecptable carrier" refers to a carrier for the administration of a therapeutic agent, comprising various excipients and diluents. The term refers to a pharmaceutical carrier that is, by itself, not essential active ingredients and is not unduly toxic after administration. Suitable carriers are well known to those skilled in the art. Pharmaceutically acceptable carriers in compositions may comprise liquids such as water, saline, buffers. In addition, auxiliary substances such as fillers, lubricants, glidants, wetting or emulsifying agents, pH buffering substances and the like may also be present in these carriers. The carrier may also comprise cell transfection reagents.

The effective amount of midbrain substantia nigradopaminergic nerve cells of the present disclosure may vary with the form of administration, the severity of the disease to be treated, and the like. Selection of preferred effective amount can be determined by those skilled in the art based on various factors (eg. through clinical trials). Such factors comprise but are not limited to: pharmacokinetic parameters, such as bioavailability, metabolism, half-life, the severity of the disease to be treated, weight, immune status of patients, the form of administration, and so on.

Specific therapeutically effective amount depends on a variety of factors, such as the particular condition to be treated, the physiological condition of the individual (eg. the weight, age or sex), the type of individual being treated, the duration of the treatment, the concurrent treatments (if any) and specific formulation being used and the structure of the compound or its derivatives. For example, a therapeutically effective amount can be expressed as the total weight of active ingredients, such as in grams, milligrams, or micrograms; or as a ratio of the weight of active ingredients to body weight, such as in milligrams per kilogram of body weight (mg/kg). Alternatively, an effective amount can be expressed as a concentration of an active ingredient (eg. cells or cell preparations of the disclosure), such as molar concentration, weight concentration, volume concentration, molar weight concentration, mole fraction, weight fraction and mixing ratio. Those skilled in the art can calculate the human equivalent dose (HED) of an agent (such as the cells or cell preparations of the present disclosure) based on the dose in animals. For example, those skilled in the art can estimate the highest safe dose for human use based on "Estimating the Maximum Safe Starting Dose in Initial Clinical Trials for Therapeutics in Adult Healthy Volunteers" published by the Food and Drug Administration (FDA) .

In a specific example of the present disclosure, some dosing regimens for animals such as mice are given. It is easy for those skilled in the art to convert the dosage of animals such as mice into dosages suitable for humans. For example, it can be calculated according to the Meeh-Rubner formula: A=k× (W^{2/3})/10,000. In the formula, A is the body surface area, calculated in m² ; W is the body weight, calculated in g; K is a constant, which varies with animal species. Generally speaking, mice and rats are 9.1, guinea pigs are 9.8, rabbits are 10.1, cats are 9.9, dogs are 11.2, monkeys are 11.8, human is 10.6. It will be understood that, depending on the drug and the clinical situation, the conversion of the administered dose may vary according to the assessment of an experienced pharmacist.

The present disclosure also provides a kit comprising the pharmaceutical composition or directly comprising the midbrain substantia nigradopaminergic nerve cells. In addition, the kit may also comprises instructions for using the medicine in the kit.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3rd ed. Science Press, 2002) or followed the manufacturer's recommendation.

### Experimental Materials

### Cell culture

H9 hESCs and reporter H9 hESCs were cultured on a feeder of irradiated mouse embryonic fibroblasts (MEFs) in the ESC medium consisting of DMEM/F-12, KOSR, 1x NEAA, 0.5x Glutamax, 0.1 mM 2-Mercaptoethanol, and 4 ng/ml FGF-2. Cells were fed daily with fresh medium and passaged weekly by Dispase II.

### Generation of Midbrain Dopamine Neurons and Forebrain Glutamate Neurons

Induction of the midbrain dopaminergic progenitors comprising: hESCs (1 day after passaging) on MEF feeder layer were cultured in the neural induction medium (NIM)(DMEM/F-12,1× NEAA, 1× N2 supplement) supplemented with SB431542 (10 µM) and DMH-1 (2 µM). To pattern the differentiating cells to the midbrain FP progenitors, SHH (C25ll, 500 ng/ml) and CHIR99021 (0.4 µM) were added to the cultures from day 1 till day 7. On day 7, individual colonies of neuroepithelial cells were gently blown off with a pipette and replated on mouse embryonic fibroblast feeder in the NIM containing SAG (1 uM) and SHH (100 ng/ml) and CHIR99021 (0.4 uM) for additional 6 days (D7-12). On day 12, CHIR99021 was removed, SHH was reduced to 20 ng/ml, SAG (0.5 µM) and FGF8b (100 ng/ml) was added to the culture to expand the progenitors in suspension till day 19. Then, 20 ng / ml SHH and 20 ng/ml FGF8b were kept in the neural induction medium till transplantation at day 32. For *in vitro* analysis, the neurospheres were dissociated by incubation in Accutase at 37°C for 3-5 minutes on day 32 and plating onto glass coverslips that were coated with Matrigel. Cells were fed with Neural differentiation medium (Neurobasal Medium, 1×N2 supplement, 1×B27 supplement) (NDM) supplemented with brain-derived neurotrophic factor (BDNF, 10 ng/ml), glial cell line derived neurotrophic factor (GDNF, 10 ng/ml), ascorbic acid (AA, 200 uM), cAMP (1 uM), transforming growth factor β3 (1 ng/ml) and Compound E (0.1 µM).

Induction of forebrain glutamate neurons from hESCs: H9 hESC colonies were cultured for 1 week with daily medium change. Then, hESC colonies were detached from the feeder layer and grown in the ES medium for 4 days to help form cell aggregates. For neural induction, cell aggregates were cultured in flasks fed with NIM supplemented with 2 µM SB431542 and 2 µM DMH-1 for 3 days. The ESC aggregates were then adhered to vitronectin coated 6-well plates in the presence of NIM till formation of neural tube-like rosettes at around day 16. The rosettes were gently blown off by a 1 mL pipette and suspended in the same medium for another 10 days. Then, progenitors were digested into small spheres with Accutase for 4 min at day 26. After an additional 1 day culture in flask with NIM, small spheres were collected and transplanted into animal models or seeded and matured in NDM supplemented with BDNF (10 ng/ml), GDNF(10 ng/ml), AA (200 µM), cAMP (1 µM), IGF1 (10 ng/ml) on glass coverslips for another 1 week for immunofluorescence staining.

### PD model and Cell Transplantation

The surgical procedure for producing the PD model was performed in SCID mice comprising: adult SCID mice (8-12 weeks) were anesthetized with 1%-2% isoflurane mixed in oxygen. 1 uL 6-OHDA (3 mg/ml, in saline with 1% ascorbic acid) was directly injected into the left substantial nigra (anterior-posterior [AP]=-2.9 mm, lateral [L]=-1.1 mm, vertical M= 4.5 mm, from skull). Animals with amphetamine-induced rotation at> 6/min over 1.5 h period were selected for cell transplantation 4 weeks after 6-OHDA-lesion surgery. Animals were randomly grouped and transplanted with glutamatergic progenitors, dopaminergic progenitors, or artificial cerebral spinal fluid (ACSF) (control). 50,000 cells were resuspended in 1 µL ACSF containing Rock inhibitor (0.5 µM), B27, 20 ng/ml BDNF and injected into the left nigra (anterior-posterior [AP]=-2.9 mm, lateral [L]=-1.1 mm, vertical [V] =4.4 mm, from skull) or left striatum (AP=+0.6 mm, L = 1.8 mm, V = 3.2 mm, from dura).

### Donor Plasmid Construction

TALEN pair, Human codon-optimized Streptococcus pyogenes wild-type Cas9 (Cas9-2A-GFP), Cas9 nickase (Cas9D10A-2A-GFP), and pCAG-Flpo (plasmid #52342, plasmid #52341, plasmid #44719, plasmid #44720, plasmid #60662) was obtained from Addgene. PL652 donor plasmid vector containing a FRT-flanked PGK-puromycin expression cassette was constructed by replacing the loxP sequence in the PL552 (#68407) with the FRT sequence.

To generate the TH-iCre donor plasmid, DNA fragments with left or right homology arm were PCR amplified from the genomic DNA immediately upstream or downstream of the STOP codon of TH gene. The DNA fragment with iCre gene fused to P2A sequence was PCR amplified from pDIRE (Addgene plasmid #80945). P2A sequences were included in the PCR primers. These three fragments were then cloned into the multiple cloning sites of plasmid PL652.

To generate TH-tdTomato donor plasmid, the DNA fragment with tdTomato gene fused to P2A was PCR amplified from pAAV-FLEX-ArchT-tdTomato (Addgene plasmid #28305). P2A sequences were included in the PCR primers. The DNA fragment with hGH polyA signal sequences was PCR amplified from AAVS1-pur-CAG-EGFP plasmid (Addgene plasmid #80945). The DNA fragments with left or right homology arm for TH-iCre donor plasmid, DNA fragment containing P2A-tdTomato sequences, and DNA fragments containing hGH polyA signal sequences were cloned into the multiple cloning sites of plasmid PL552.

To generate AAVS1-neo-CAG-ChR2-EYFP donor plasmid, the inventors replaced the FlpeERT2 gene in the AAVS1-Neo-CAG-Flpe-ERT2 plasmid (Addgene plasmid #68460) with ChR2-EYFP gene which is PCR amplified from pAAV-hSyn-hChR2 (H134R)-EYFP (Addgene plasmid #26973).

To generate the AAVS1-pur-CAG-Bi-DREADD donor plasmid (AAVS1-pur-CAG-hM3Dq-mcherry-P2A-HA-KORD) or AAVS1-pur-CAG-mCherry donor plasmid, the inventor amplified mCherry or hM3Dq-mCherry from AAVS1-pur-CAG-hM3Dq-mCherry (Addgene plasmidl #80948), HA-KORD from pAAV-hSyn-dF-HA-KORD-IRES-mCitrine (Addgene plasmid #65417). The two DREADD genes, hMsDq-mcherry and HA-KORD, were linked by P2A peptide (hM3Dq-mcherry-P2A-HA-KORD) to ensure simultaneous expression of these two genes in the same cells. The hMaDq-mcherry-P2A-HA-KORD or mCherry gene was inserted into the AAVS1-pur-CAG-EGFP to replace EGFP. SA-Neo, splice acceptor sequence followed by T2A self-cleaving peptide sequence, and then the neomycin resistance gene. CAG, synthetic CAGGS promoter with the actin enhancer and the cytomegalo-virus early promoter.

### Electroporation and Generation of Reporter hESC Lines

H9 hESCs were pretreated with Rho-kinase (ROCK) inhibitor for 6-8 h (0.5 mM). Cells were then digested by TrypLE^{™} Express Enzyme, dispersed into single cells, and electroporated with appropriate plasmids in 500 mL of electroporation buffer (5 mM KCI, 5 mM MgCI₂, 15 mM HEPES, 102.94 mM Na₂HPO₄, and 47.06 mM NaH₂PO₄, pH=7.2) using the Gene Pulser Xcell System (Bio-Rad) at 250 V, 500 mF in a 0.4 cm cuvette (Phenix Research Products). Cells were then seeded on MEF feeder layer in 6-well plate in MEF-conditioned medium with ROCK inhibitor. Cells were fed daily with MEF-conditioned ESC medium (CM). 72 hours later, puromycin (0.5 µg/ml) or G418 (50-100 µg/ml) were added into CM for selection for two weeks. After selection, cells were pretreated with ROCK inhibitor for 6-8 h, and then individual clones were picked up. Genomic PCR was applied to examine the integration of the transgene.

For generation of TH-iCre knock-in hESC line, the cassette containing a P2A peptide sequence-linked codon-improved Cre recombinase (iCre) gene with a STOP codon and then FRT-flanked PGK-Puro sequence (PGK promoter-driven) was introduced to immediately upstream of the STOP codon of the endogenous TH gene of H9 ESCs. FRT-flanked PGK-Pur was then removed by transient expression of Flpo.

For generation of TH-tdTomato/AAVS1-ChR2-EYFP hESC line, the cassette containing a P2A peptide sequence-linked tdTomato gene with STOP codon followed by polyA sequence and then PGK-Pur sequence was introduced to immediate upstream of the STOP codon of the endogenous TH gene of H9 ESCs by CRISPR, and then the ChR2 expression cassette was knocked into the AAVS1 locus by TALEN. For generation of Bi-DREADD or mCherry hESCs, the hMDq-mCherry-P2A-HA-KORD or mCherry expression cassette was inserted into the AAVS1 locus of H9 hESCs using TALEN.

### Whole-Cell Patch-Clamp and Brain Slice Recording

Coronal brain slices (350 um thick) at the level of the forebrain or the midbrain were prepared from recovered animals at 3 and 6 months post-transplantation using a vibratome (Leica VT1200S) in ice-cold cutting solution (100 mM glucose, 75 mM NaCI, 26 mM NaHCO3, 2.5 mM KCI, 2 mM MgCI2-6H2O, 1.25 mM NaH2PO4-6H20, and 0.7 mM CaCI2). The slices were transferred to the recording artificial cerebrospinal fluid (ACSF, 124 mM NaCI, 4.4 mM KCI, 2 mM CaCI2, 1 mM MgSO4, 25 mM NaHCO3, 1 mM NaH2PO4, and 10 mM glucose) saturated with 95% O2/5% CO2. Voltage and current signals were recorded by Axon 700B amplifier (Axon). The recording electrodes (3-5 MΩ) were filled with a solution containing 112 mM Cs-Gluconate, 5 mM TEA-CI, 3.7 mM NaCI, 0.2 mM EGTA, 10 mM HEPES, 2 mM MgATP, 0.3 mM Na3GTP and 5 mM QX-314 (adjusted to PH 7.2 with CsOH) for spontaneous excitatory post-synaptic current (sEPSC) and spontaneous inhibitory post-synaptic current (sIPSC) recording. For sEPSC or sIPSC recording, cells were voltage clamped at 60 mV or 0 mV, respectively. The initial access resistance was monitored throughout the experiment, ranging from 15-30 MΩ. Cells with the access resistance changed > 15% were discarded. Data were filtered at 1 kHz and digitized at 10 kHz. Action potentials (APs) in response to the blue light stimulation (473 nm, frequency 5 Hz, intensity 10 mM/mm2) or the depolarizing currents (0-100 pA, step 10 pA, duration 2 s) were recorded in current clamp mood. Sag measurement was conducted under current clamp mode by injection of 90 pA or 120 pA currents into the grafted or endogenous mDA neurons, respectively. Transplanted mDA neurons and non-mDA neurons were identified by their tdTomato fluorescence in the EYFP-positive graft.

### Viral Injections and Rabies Tracing Experiments

For rabies tracing experiments, 200 nL AAV expressing Cre-dependent TVA and tdTomato (AAV2 / 9-Ef1a-DIO-TVA-2A-NLS-tdTomato, titer 1.29^{∗}10^12 genome copies (gc)/ ml ), or 200 nL AAV expressing Cre-dependent Rabies Glycoprotein (AAV2 / 9-Ef1a-DIO-G, titer 1.29^{∗}10^12gc / ml) were co-injected into the graft site (For nigral graft: AP=-2.9 mm, L=1.1 mm, V=4.4 mm, from skull; For striatum graft: AP= +0.6 mm, L= -1.8 mm, V= 3.2 mm, from dura) of PD mice 5 months after transplantation. 3 weeks later, EnVA-pseudotyped, rabies G deleted, EGFP-expressing rabies virus (RVdG-EGFP, 400 nl, titer 2^{∗}10^8pfu / ml) was injected into the same site for trans-synaptic labeling. One week later, thel mice were sacrificed for histological analysis. For endogenous mDA neuron, viruses were injected to the SNc (AP= 2.9 mm, L= 1.1 mm, V= 4.5 mm, from skull) of DAT-Cre / Ai9 mice. After fixation, the brain was sectioned (30 um thick) with a freezing microtome. All coronal sections (1:4 series) without staining were imaged by a 20x objective with a fluorescence microscope (Olympus VS120). Tiled images were automatically stitched using a 10% overlap with VS-ASW (Olympus) software. The locations of labeled neurons and the outlines of brain areas were manually labeled using Photoshop according to Paxinos and Franklin (2007). Some sections underwent immunostaining to elucidate cell identity.

### Tissue Preparation and Immunohistochemistry

Animals were sacrificed with an overdose of pentobarbital (250 mg/kg, i.p.) and perfused with saline followed by 4% ice-cold phos-l phate-buffered paraformaldehyde (PFA). The brains were removed and immersed sequentially in 20% and 30% sucrose until sunk. Serial sagittal (0.12 to 3.12 mm from medial to lateral) or coronal (1.42 to 0.10 mm from the Bregma) sections were cut on a freezing microtome (Leica SM2010R) at a thickness of 30 mm and stored at 20°C in a cryoprotectant solution. Free-floating sections were incubated with a primary antibody in 4°C for 1-2 nights, and then the unbound primary antibodies were removed. For DAB staining, sections were incubated with corresponding biotinylated secondary antibodies for 1h followed by avidin-biotin peroxidase for 1h at room temperature. Immunoreactivity was visualized with DAB staining kit. The sections were then dehydrated with ethanol, permeabilized in xylene, and mounted in neutral resin. For fluorescent immunolabeling, sections were incubated with corresponding fluorescent secondary antibodies for 1h at room temperature. Then sections were mounted by Fluoromount-G.

### Packaging of Lentivirus

Lentiviruses were generated in 293T cells by transfecting packaging and backbone plasmids using calcium phosphate/DNA copre-cipitation method. 293T cell were cultured in Dulbecco's MEM (DMEM) containing 10% FBS. The supernatant containing the viral particles was collected 72 hours after transfection, and concentrated by ultracentrifugation at 27000 rpm for 2 hours at 4°C. The viral particles were then resuspended in DPBS.

### Imaging and Cellular Quantification

To quantify the population of EN1, FOXA2, LMX1A, NURR1, GIRK2, and TUJ-1 expressing cells among total TH or TH to total cells, at least five randomly chosen images from coverslips were counted with ImageJ software. Data were replicated three times and were expressed as mean ± SEM. For measuring the human fiber density in the brain slices, tiled images were captured by a Nikon TE600 or Olympus VS120 microscope. The optical density of human fibers in different areas of the mouse brain was measured by image processing and analysis system (Image Pro Plus 5.1 software). Data were shown as optical density in different areas. For TH, GIRK2, LMX1A, human nuclei (hN) and FOXA2 staining, the graft was outlined and captured by a 60x objective with Nikon A1R-Si laser-scanning confocal microscope (Nikon, Tokyo, Japan) or a fluorescence microscope (Olympus VS120). Single or double stained cells were counted manually with ImageJ. Data were presented as ratio of TH-, LMX1A-, FOXA2- to total hN or GIRK2/TH/hN to TH/hN cells. All data are expressed as mean ± SEM.

### Behavioral Test: Rotation Test

Amphetamine-induced rotations were tested before transplantation and every month till 6 months after transplantation. Rotation was recorded by a video camera for 1.5 h, 5-10 min after peritoneally amphetamine (2 mg/ml in saline, 5 mg/kg) injection. Data were presented as the average net number of rotations per minute during 90 min.

For spontaneous rotation test, animals were recorded for 60 min after injection of CNO (1.2 mg/kg) for 20 min, SALB (5 mg/kg) for 5 min, or saline for 20 min.

### Behavioral Test: Cylinder Test

Individual animal was placed in a glass cylinder and recorded by a camera for 3 min. The ipsilateral and contralateral paw touches to the wall of the cylinder were counted. The data were expressed as the percentage of ipsilateral touches to total touches. For drug treatment, animals were treated by CNO (1.2 mg/kg) for 20 min, SALB (5 mg/kg) for 5 min, or saline for 20 min before Cylinder test.

### Behavioral Test: Rotarod Test

An accelerating Rotarod (Med Associates Instruments) was used to test motor coordination. All animals were pre-trained for two days in order to reach a stable performance. On day 1, mice were trained on a rotating rod that accelerated from 2 per minute (rpm) to 20 rpm in a period of 300 s for three times. On day 2, mice were trained on rod accelerated from 3 rpm to 30 rpm twice, and from 4 rpm to 40 rpm once, in a period of 300 s. The test was performed from the third day on a rotating rod that accelerated from 4 rpm to 40 rpm in a period of 300 s. The period of time the mouse stayed on the rod was monitored. The average duration from three repeated tests of each animal was used for data analysis.

### Quantification and statistical analysis

SPSS software was used for statistical analysis. In all studies, data were analyzed by Student-t test, Paired t test, two-way ANOVA followed by Holm-Sidak test, Two-way RM ANOVA followed by Tukey's post hoc test, or One-way ANOVA followed by Holm-Sidak test. Statistical significance was determined at p < 0.05.

English notes of all abbreviation in the present disclosure are shown in Table 1.

**Table 1**

| | | | |
|---|---|---|---|
| Acb | Accumbens nucleus | Pa | Paraventricular hypothalamic nucleus |
| AcbC | Accumbens nucleus core | PAG | Periaqueductal gray |
| AcbSh | Accumbens nucleus shell | PB | Parabrachial nucleus |
| AI | Agranular insular cortex | PF | Parafascicular thalamic nucleus |
| Amy | Amygdala | PLH | Peduncular part of lateral hypothalamus |
| AOV | Anterior olfactory nucleus, ventral part | Pn | Pontine reticular nucleus |
| APT | Anterior pretectal nucleus | PO | Preoptic area |
| ASt | Amygdala striatal transition area | PrCnF | Precuneiform area |
| Ce | Central nucleus of the amygdala | SI | Substantia innominata |
| CPu | Caudate putamen | SN | Substabtia nigra |
| DR | Dorsal raphe nucleus | SNC | Substantia nigra, pars compacta |
| DTT | Dorsal tenia tecta | SNR | Substantia nigra, pars reticularis |
| EA | Extended amygdala | ST | Bed nucleus of the stria terminalis |
| FrA | Frontal association cortex | STh | Subthalamic nucleus |
| G | Geniculate nucleus | | |
| GP | Globus pallidus | Tu | Olfactory tubercle |
| Hipp | Hippocampus | VM | Ventromedial thalamic nucleus |
| HT | Hypothalamus | VMH | Ventromedial hypothalamic nucleus |
| IPAC | Interstitial nucleus of the posterior limb of the anterior | VP | Ventral pallidum |
| LPO | Lateral preoptic nucleus | VS | Vetral striatum |
| M | Motor cortex | ZI | Zona incerta |
| M1 | Primary motor cortex | | |
| M2 | Secondary motor cortex | | |
| MD | Mediodorsal thalamic nucleus | | |
| MFB | Medial forebrain bundle | | |
| MnR | Median raphe nucleus | | |
| MPA | Medial preoptic area | | |
| mRt | Mesencephalic reticular formation | | |
| OB | Olfactory bulb | | |

### Example 1. Grafted Human mDA and Glu Neurons Project to Differential Targets

During development, targeted axonal projection is often determined by the intrinsic properties of the cells. To address whether cell-intrinsic properties also determine target finding in the adult brain, the inventors transplanted hESC-derived mDA or forebrain Glu neuron progenitors into themidbrain of PD model mice. mDA or Glu neuron progenitors were differentiated from hESCs according to the protocols of the inventors. On day 32 of mDA neuron differentiation (the day of transplantation), most of the progenitors expressed the floor plate and midbrain markers CORIN, FOXA2, LMX1A, and EN1 (Figures 8A and 8C). By day 42, 69% of total cells or 84% of TUJ1+ neurons were positive for tyrosine hydroxylase (TH) as well as EN1, FOXA2, LMX1A, and NURR1 (Figures 8D and 8F), suggesting an mDA neuronal identity. Furthermore, most TH+ neurons co-expressed GIRK2 (>85%), a marker relatively enriched in A9 mDA neurons, but fewer expressed Calbindin (CALB) (15%), a marker expressed in A10 mDA neurons (Figures 8D and 8F). Thus, most of the TH+ neurons carry A9 mDA neuron characteristics. On day 32 of Glu neuron differentiation, most cells expressed the dorsal forebrain marker PAX6 and the forebrain marker FOXG1, demonstrating their dorsal forebrain progenitor identity (Figures 8B and 8C). By day 42, 82% of total cells became positive for vGLUT1, and most of these neurons expressed CTIP2 (>80%) or TBR1 (>85%), suggesting a forebrain layer 5/6 cortical Glu neuron identity (Figures 8E and 8F).

The inventors then transplanted the mDA (Figure 1A) or Glu progenitors (Figure 1B) into the SN of PD mice. Six months following transplantation, grafts were present in all grafted animals. Serial sagittal sections of the mDA neuron-transplanted brain revealed that most hNCAM+ fibers were distributed in the caudate putamen (CPu) (Figures 1A and 1D), the area mainly innervated by A9 mDA neurons (Bjorklund and Dunnett, 2007). Quantification of hNCAM fiber density from three representative sagittal planes (Figure 1C) showed that 72% of total hNCAM+ fibers in plane L2.16, 62% in plane L1.44, and 45% in plane L0.72 were distributed in the CPu (Figure 1E). Fewer hNCAM+ fibers were detected in the olfactory tubercle (Tu;17%-20%) and accumbens nucleus (Acb; 9%-16%), areas targeted by A10 mDA neurons. The remaining areas, including the amygdala and cortex, accounted for less than 15% of total fibers (Figure 1E). Within the striatum, 72% and 87% of human fibers projected to the dorsal striatum (CPu) in planes L0.72 and L1.44, respectively (Figure 1F). The distribution pattern of hNCAM + fibers, especially within the CPu, is similar to that of endogenous TH + fibers in normal animals (Figures 1D and 8G).

In contrast, Glu neurons extended axons locally, filling theentire midbrain (Figure 1B). They also sent out axons over a long distance, but mostly to the amygdala, olfactory bulb (OB), substantia innominata (SI), and cerebral cortex, with only a small portion of hNCAM+ fibers (2%-8%) detected in the CPu (Figures 1B, 1D, and 1E).

Together, these results indicate that the grafted human neural progenitors differentiate to respective neuronal types and project axons to different brain regions.

### Example 2. Grafted Human mDA Neurons Project through Their Cognate Path

Examination from serial sagittal sections (Figure 2A) revealedthat the grafted mDA neurons extended axons in the rostral direction along the medial forebrain bundle (MFB) in a well-defined and fasciculated tract (Figure 2B, L1.08, and red arrowheads in Figure 2C). Laterally, they were seen to extend through the SI (Figure 2B, L1.44) and amygdala (Figure 2B, L2.04). Rostrally, hNCAM+ fibers penetrated the Acb (Figures 2B-2E), where alarge portion of hNCAM+ fibers ascended into the CPu along the boundary between the cortex and striatum (Figure 2A and red arrows in Figure 2D). A few hNCAM+ fibers were detected in the cortex (blue arrowheads in Figures 2D and 2F). These results suggest that most of the axonal projections from grafted mDA neurons follow the endogenous nigra-striatal pathway.

In the dorsal/lateral striatum, the dense hNCAM fibers exhibited elaborately ramified, fine-beaded terminal networks (Figures 2D-2F and Figure 9A). Ramification of the axons was also observed in the Tu (Figures 2C and 2D) but not in the Acb, amygdala, and MFB (Figure 9B), suggesting target-specific axonal branching. Most of the human fibers, stained by STEM121, were positive for TH (Figures 2G and 2H), demonstrating a dopaminergic identity. Ramification of hNCAM+ fibers from a Glu neuron graft was observed in the OB and cortex (Figure 9C).

Immunohistochemistry analysis of the mDA graft showed that 68% of the grafted cells co-expressed TH and human nuclei (hNs), and most of the TH+ cells also expressed GIRK2 as well as FOXA2 and LMX1A (Figures 9D-9F), suggestive of A9 mDA identity. Human-specific synaptophysin (hSyn) puncta were distributed along TH+ fibers in the CPu, and some were localized on the GABA+ cell body (Figure 9G). In contrast, few hSyn+ puncta were observed in the forebrain neuron-grafted group, and hardly any of them were localized on the GABA+ cell body (Figure 9H). These results suggest synaptic connections between grafted human mDA neurons and target cells in the host brain.

### Example 3. Genetic Labeling Reveals Specific Axonal Innervation by Human mDA Neurons

To elucidate the specific axonal innervation by grafted mDA neurons, we created a TH reporter hESC line with tdTomato expres-sion recapitulating that of the endogenous TH gene (Method Details). The inventors further knocked in a ChR2-EYFP fusion protein expression cassette into the AAVS1 locus to enable specific labeling and manipulation of transplanted human cells (Figures 3A, 3B, 10A, and 10B). The final hESCs, called TH-tdTomato/AAVS1-ChR2-EYFP hESCs, constitutively expressed ChR2-EYFP during the entire mDA neuron differentiation process, whereas tdTomato was expressed only at the later stages of mDA neuron differentiation (Figure 10C) and exclusively in TH+ mDA neurons (Figures 3C and 10D), highlighting the specific reporting of TH+ cells.

We then transplanted the mDA progenitors derived from TH-tdTomato/AAVS1-ChR2-EYFP hESCs into the SN or striatum of PD model mice (Figure 3A). Six months after transplantation, EYFP+ grafts were present in all grafted animals (Figure 3D), and tdTomato was only expressed in TH+ mDA neurons but not non-mDA neurons; e.g., serotonin (5-hydroxytryptamine, 5-HT) neurons (Figures 10E and 10F). Serial coronal sections ofthe brain with the SN graft showed that most human mDA neuron projections were distributed in the CPu, where fibers formed dense and ramified networks (Figures 3E, b and c). Only a small proportion of mDA neuron projections was present in the Acb (Figure 3E, slices 1-3). The rostral ascending projection path of mDA neuron axons was identified in these coronal sections (Figure 3E, slices 1 and 2, a and b, dotted red arrows). tdTomatot projections were positive for STEM121 (Figure 3F), confirming their human identity. These results verify the specific axonal pathfinding and targeting from grafted human mDA neurons shown by hNCAM staining (Figures 1 and 2). In the striatal graft, tdTomato+ mDA neuron fibers occupied the entire CPu, and most of the fibers were confined to the CPu (Figures 3G, 3H, and 10G). In the nigral graft, tdTomato+ fibers displayed abundant dendritic outgrowths limited to the surrounding graft (Figure 3l), suggesting cell- and target-specific ramification of human mDA dendrites and axons. In addition, hESC-derived dopamine (DA) neurons were distributed in the periphery of the striatal and nigral grafts (Figures 3H and 3I), a phenomenon strikingly similar to that of human fetal grafts in PD patients.

The cellular features (Figures 8D, 9D, and 9F) and the specific projection pattern (Figures 1, 2, and 3) suggest that our mDA neurons resemble those in the SN pars compacta (SNc). Taking advantage of the genetic reporter, we performed whole-cell patch-clamp recording on mDA neurons (EYFP+/tdTomato+) and non-mDA neurons (EYFP+/tdTomato+) (Figure 10H). The inventors found that human mDA neurons and non-mDA neurons in the striatal or nigral grafts displayed current-or blue light-induced action potentials (APs) and spontaneous APs (sAPs) by 3 months after transplantation (Figures 3J and 11A-11F), suggesting functional maturation of grafted human neurons. Importantly, the SAPs of human mDA neurons grafted in the striatum and nigra displayed regular discharge at a low spiking rate (0.87 ±0.20 Hz for the striatal group, 0.83 ± 0.15 Hz for the nigral group) and slow sub-threshold oscillatory potentials (0.29 ± 0.06 Hz for the striatal group, 0.26 ± 0.13 Hz for the nigral group) (Figures 3J, 11G, and 11H). Prominent afterhyperpolarization (AHP) was observed in the sAP of human mDA neurons 6 months after transplantation (Figures 11I and 11J). These physiological features are consistent with characteristics of endogenous SNc (A9) mDA neurons (Figure 3J; Guzman et al., 2009; Lammel et al., 2008; Nedergaard et al., 1993). In addition, grafted mDA neurons displayed a higher membrane capacitance (Cm) and a trend of lower neuronal input resistance (Rm) compared with non-mDA neurons in the striatal or nigral graft 6 months after transplantation (Figures 11K and 11L). Furthermore, endogenous SNc mDA neurons are characterized by sag potentials in response to a hyperpolarizing current injection (Evans et al., 2017; Lammel et al., 2008; Neuhoff et al., 2002). Using mDA neuron reporter mice (dopamine transporter (DAT)-Cre/Ai9), the inventors found that all of the recorded SNc neurons displayed a typical sag component in the subthreshold range (37.2 ± 3.8 mV, n =15/15) (Figures 3K and 3M). However, only 5 of 11 endogenous medial ventral tegmental area (VTA) mDA neurons showed a sag component, whose amplitude was much smaller (23.0±1.6 mV, n = 5/11) (Figures 3K and 3M). Interestingly, grafted human mDA neurons in the striatum displayed a pronounced sag component (32.5 ± 3.3 mV, n = 13/16). In contrast, only 11 of 29 grafted non-mDA neurons showed a sag component with a much smaller amplitude (14.9 ± 2.9 mV, n = 11/29) (Figures 3L and 3M).

Together, these results indicate that grafted human mDA neurons have functional characteristics of A9 mDA neurons.

### Example 4. Anatomical Synaptic Inputs to Human Neurons Are Associated with Transplant Sites

Rabies-mediated tracing has been used to track anatomical inputs onto transplanted cells in the PD model. To reveal presynaptic inputs specifically to grafted human mDA neurons, the inventors combined the Cre-loxP gene expression system with rabies-mediated transsynaptic tracing (Figure 4A). The inventors created a TH-iCre hESC line, allowing Cre recombinase expression directed to TH-expressing cells without disrupting endogenous TH expression (Figures 4B, 12A, and 12B). The specificity of this system was evidenced by exclusive mCherry expression in TH+ mDA neurons following infection of TH-iCre hESC-derived neuronal culture with a lentivirus expressing Cre-dependent mCherry (Figures 12C and 12D). Five months following transplantation of TH-iCre mDA progenitors into the nigra or striatum of PD mice, an adeno-associated virus (AAV) expressing Cre-dependent (DIO, or double-floxed inverted orientation) TVA and tdTomato with nuclear location signal (NLS-tdTomato) (AAV-DIO-TVA-2A-NLS-tdTomato) as well as an AAV expressing a Cre-dependent rabies glycoprotein (G) (AAV-DIO-G) were co-injected into the graft sites (Figure 4A). One month later, the EnvA-pseudotyped and G-deleted rabies virus expressing EGFP (RVdG-EGFP) was injected into the graftsites (Figure 4A). Because only grafted human mDA neurons express Cre recombinase, expression of TVA, tdTomato, and G is restricted to human mDA neurons, not non-mDA neurons. Indeed, tdTomato was expressed only in TH+ human mDA neu-rons (Figure 12E). Because RVdG-EGFP only infects TVA-expressing (tdTomato+) human mDA neurons, the grafted starter human mDA neurons co-express EGFP and tdTomato. Coexpression of G in grafted human mDA neurons enabled RVdG-EGFP to spread transsynaptically to their presynaptic partners (Wickersham et al., 2007); hence, host presynaptic neurons express EGFP only (Figure 4A).

The starter neurons (EGFP+/tdTomato+) were only found in human grafts, and they were TH+ (Figures 4C and 4D). Transsynaptically labeled host neurons (EGFP+/tdTomato+) were readily detected in brain regions away from the graft (Figure 4E). In the nigrally grafted brain, the most abundantly labeled host neurons were found in the striatum, including the CPu and Acb (Figures 4E-4G). The labeled host neurons in the CPu expressed GABA and DARPP32, suggesting striatal medium spiny neurons (Figure 12F). In the Acb, the labeled host neurons formed patches, which was consistently observed in different samples (Figure 4H). In the cortical area, CTIP2+ and SATB2+ cortical neurons were found to project to nigral human mDA neurons (Figures 4E-4G and 12F). In the hypothalamic area, the peduncular part of the lateral hypothalamus (PLH) and the paraventricular hypothalam-ic nucleus (Pa) projected strongly to human mDA neurons in the SN. Densely labeled host neurons were also found in the bed nu-cleus of the stria terminalis (ST) and the central amygdala nucleus (Ce) but not other amygdala regions (Figures 4E-4G). In addition, scattered neurons were observed in the globus pallidus (GP), ventral pallidum (VP), and extended amygdala (EA). In more caudal regions, the dorsal raphe (DR), periaqueductal gray (PAG) and pontine reticular nucleus (Pn) contained largenumbers of labeled neurons (Figures 4E-4G). The inventors identified 5-HT+ serotonin neurons in the DR projecting to human mDA neurons in the SN (Figure 12F). The distribution of presynaptic inputs to the nigrally grafted human mDA neurons was strikingly similar to that onto the endogenous SNc mDA neurons in DAT-Cre mice, which express Cre in DA neurons (Figures 12G and 12H), and a patch-like distribution of inputs to endogenous mDA neurons was also observed in the Acb.

In the brain with the striatal graft, dense presynaptic inputs were found in the CPu but not the Acb. More inputs were observed in the GP and cortical areas compared with those to nigrally grafted human mDA neurons. The parafascicular thalamic nucleus (PF) and mediodorsal thalamic nucleus (MD) preferentially projected to striatally grafted human mDA neurons. Labeled host neurons were also found in the Ce and SN reticular part (SNR) in the brain with a striatal graft. Few labeled neuronswere found in the more caudal brain regions, such as the PAG, DR, parabrachial nucleus (PB), and Pn (Figures 4E, 4F, and 12J).

Thus, human mDA neurons grafted in the striatum and nigra receive inputs from largely different brain regions, suggesting location-dependent presynaptic inputs. Nigrally grafted human mDA neurons receive extensive inputs from similar brain regions, as endogenous mDA neurons do.

### Example 5. The Identity of a Grafted Neuron Determines Its Functional Input Characteristics

Electrophysiological recording showed that few sEPSCs and sIPSCs (spontaneous excitatory and inhibitory postsynaptic currents, respectively) were detected in human mDA and non-mDA neurons in striatal or nigral grafts 3 months after transplantation (Figures 5A-5D). Strikingly, the mean frequency, but not the amplitude, of sIPSCs and sEPSCs from mDA and non-mDA neurons in the striatal or nigral grafts were increased significantly 6 months after transplantation (Figures 5A-5D). No differences in sEPSC or sIPSC rise time or decay time between grafted non-mDA and mDA neurons were observed (Figures 13A-13F). These results demonstrate that the functional inputs are established during the 3-to 6-month period, which is not affected by graft locations or neuronal types.

Interestingly, the sIPSC frequency in mDA neurons was higher than that in non-mDA neurons in the nigral grafts (Figure 5C). In contrast, the sEPSC frequency in mDA neurons was significantly lower than that in non-mDA neurons in striatal and nigral grafts (Figure 5D). By calculating the sIPSC/sEPSC ratio, we found that nigrally grafted human mDA neurons receive more inhibitory inputs with a sIPSC/sEPSC ratio of 3.28, a pattern similar to that of endogenous mDA neurons, whereas human non-mDA neurons received almost equal inhibitory/excitatory inputs, with a sIPSC/sEPSC ratio of 0.95 (Figures 5E and 13G). A trend of a higher sIPSC/sEPSC ratio (2.61) of human mDA neurons compared with non-mDA neurons was also observed in striatal grafts (Figure 5E), whereas endogenous striatum neurons received more excitatory inputs with a sIPSC/sEPSC ratio of 0.86 (Figures 5E and 13H).

These results suggest that the identityof the grafted neurons, rather than the graft site, determines the inhibitory and excitatory input characteristics of the grafted neurons.

### Example 6. Transplantation of mDA but Not Glu Neurons Corrects Motor Deficits in PD Mice

The functional effect of the nigral grafts was assessed by amphetamine-induced rotation, rotarod test, and cylinder test before and every 4 weeks after grafting (Figure 6A). PD mice with the striatal graft began to recover at 3 months and recovered 4 months post-transplantation in amphetamine-induced rotation. Over time, the mice gradually exhibited overcompensation by rotating to the contralateral side (Figure 6B). Functional recovery was also observed in PD mice with a nigral mDA graft 4-5 months later; however, no overcompensation was detected up to 6 months (p<0.001; Figure 6B). In contrast, those that received nigral Glu neurons or artificial cerebrospinal fluid (ACSF; controls) did not show any sign of recovery in amphetamine-induced rotation (p > 0.05; Figure 6B).

In the rotarod test, which is used to assess motor coordination and balance and does not depend on pharmacological stimulation of the dopaminergic system, the latency to fall was significantly increased over time in PD mice that received nigral orstriatal transplantation of human mDA neurons (p < 0.001) but not in those that received nigral Glu neurons or ACSF (p >0.05; Figure 6C).

In the cylinder test, a measure of forelimb akinesia, all groups presented preferential ipsilateral touches after 6-OHDA lesion. The ipsilateral touching preferences were reduced significantly (close to 50%) 4 months after nigral or striatal transplantation of human mDA-rich neurons (p < 0.001) but not nigral Glu neurons or ACSF (p> 0.05; Figure 6D).

### Example 7. Motor Recovery Depends on Functional Reconstruction of the Nigra-Striatal Circuit

To determine whether behavioral recovery of PD-model animals depends on the reconstructed nigra-striatal circuit, we transplanted mDA neurons that were derived from hESCs with a "bi-directional switch"(Figure 7A). The hESC line, called Bi-DREADD-hESCs, was established by inserting into the AAVS1 locus two DREADDs (designer receptors exclusively activated by designer drugs), an excitatory hM3Dq activated by clozapine-N-oxide (CNO) (Alexander et al., 2009) and an inhibitory KORD activated by Salvinorin B (SALB) (Vardy et al., 2015; Figures 7B, 14A, and 14B). hESCs with an mCherry expression cassette knocked into the AAVS1 locus (mCherry-hESCs) were included as a control (Figures 7B, 14A, and 14B). Expression of the transgenes was readily detected in neurons derived from Bi-DREADD-hESCs or mCherry-hESCs in culture or after transplantation (Figures 7C, 7D, and 14C-14G). Six months after transplantation, motor recovery was observed in the Bi-DREADD group and them Cherry (control) group, as evidenced by reduced amphetamine-induced rotation and reduced ipsilateral touching pref-erences (p< 0.05; Figures 7E-7G).

Using the cylinder test and spontaneous rotation test, which do not require stimulation of DA release by amphetamine, we found that CNO (1 mg/kg) treatment further decreased the preferential ipsilateral touches (p <0.05; Figure 7H), whereas SALB (5 mg/kg) treatment increased the ipsilateral touches in the same animals with the Bi-DREADD graft (p <0.01; Figure 7H). CNO or SALB treatment had no effect on ipsilateral touches in mice receiving an mCherry-expressing graft (p > 0.05; Figure 7H). In the spontaneous rotation test, vehicle-treated mice did not show obvious motor balance changes. However, CNO treatment significantly induced more contralateral than ipsilateral rotations (p < 0.01) in mice transplanted with Bi-DREADD cells (Figure 7I). This is better shown by the ipsilateral rotation ratio from 48.49 ± 8.10 to 34.83 ± 6.23 (p<0.05; Figure 7J). In contrast, SALB significantly increased ipsilateral net rotations(p< 0.05), or an ipsilateral rotation ratio of 58.42 ± 9.91 from 48.49 ± 8.10 (p< 0.01; Figure 7J). mCherry mice did not show obvious changes with or without CNO or SALB treatment (p > 0.05; Figures 7I and 7J).

These results suggest that recoveryof forelimb akinesia and asymmetric rotation depends on graft activity.

### Discussion

This disclosure developed genetic labeling strategies to precisely map the projection from and synaptic inputs to grafted human mDA neurons in a mouse model of PD. The inventors found that human mDA neurons transplanted into the nigra specifically projected to the dorsal striatum. The grafted mDA neurons receive synaptic inputs, as revealed by rabies-mediated tracing, in a pattern strikingly similar to those to endogenous mDA neurons. Electrophysiological recording revealed predominantly inhibitory inputs to the grafted mDA neurons, which appears to be dependent on cell identity but not transplant site. With pre- and post-synaptic integration, homotopically grafted human mDA neurons rescue the motor deficits of PD mice in a manner dependent on graft activity. These findings reveal cell-type-dependent functional circuit integration by transplanted neurons, highlighting the prospect of using specialized neuronal types from stem cells to repair the neural circuit to treat neurological conditions.

What determines the targeted projection by grafted neurons in the mature brain remains unknown. By transplanting two types of projection neurons, mDA and Glu neurons, into the SN of PD mice, the inventors found that both neuronal types project axons over a long distance, but to different targets, via distinct routes, with the majority of axons of grafted mDA neurons targeting the CPu. This is further verified by using TH reporter cells, showing nearly exclusive projection to the dorsal (CPu) but not ventral striatum (Acb). Because the CPu is the main target of SNc (A9) mDA neurons (Björklund and Dunnett, 2007; Joel and Weiner,2000), the inventors' finding suggests that human mDA neurons are mostly A9-like cells. Indeed, cellular characterization of the inventors and, in particular, electrophysiological recording confirm an A9 identity of the human mDA neurons. This interpretation suggests that many of the axonal projections to other brain regions seen in the previous studies may be coming from non-mDA neurons. Together, these results strongly suggest that pathfinding and target projection are largely determined by the identity of the grafted neurons.

Correct synaptic inputs into grafted neurons are also critical for restoration of lost function. In the present disclosure, the TH-iCre system specifically enables identification of monosynaptic inputs to grafted human mDA neurons, revealing an interesting pattern. By looking at the graft in general, the anatomical synaptic inputs seem to be associated with the graft sites even though nigrally and striatally grafted mDA neurons receive inputs from overlapping areas, like the dorsal striatum, similar to the observationmade by Adler et al. However, the striking similarity of the inputs to nigrally grafted human mDA neurons and endogenous mDA neurons suggests that cell identity plays a role in dictating synaptic inputs. This is displayed more clearly at the functional level. Grafted mDA neurons receive more inhibitory but less excitatory inputs compared with non-mDA neurons, regardless of whether the cells are transplanted into the striatumor nigra, suggesting that the identity of the grafted neurons dictates the functional synaptic inputs.

With pre- and post-synaptic integration by transplanted mDA neurons, it is natural to believe that the reconstructed nigra-striatal circuit contributes to motor recovery of PD mice. It has been shown that human mDA neurons transplanted into the striatum functionally connect with striatal neurons and contribute to animal behavior recovery using optogenetic and chemogenetic tools. In the current study, use of the Bi-DREADD strategy, which enables excitationand inhibition on the same grafted cells, clearly demonstrates that the reconstructed nigra-striatal circuit is functional, underlying the behavior recovery of PD mice.

Taken together with precise genetic labeling and functional measurements, we revealed that restoration of a neural circuit by transplanted cells in the mature brain, including pathfinding, targeting specificity, and functional input establishment, is largely determined by the intrinsic properties of the grafted neurons. Therefore, it is critical to transplant highly enriched, appropriately fated neural progenitors to achieve reconstruction of specific circuits for therapeutic outcomes. Hence, cell-based therapy to treat neurological conditions is realistic.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A method for preparing midbrain substantia nigra dopaminergic neurons, comprising:
(1) Culturing stem cells in a medium containing neural induction agents, ie. supplementing components in consecutive multiple stages to accomplish induction; and
(2) Obtaining the stem cell-derived midbrain substantia nigra dopaminergic neurons from the culture.

2. The method according to claim 1, wherein in (1), said multiple stages for induction with supplementary components are:
First stage: Adding SB431542, DMH-1, SHH and CHIR99021;
Second stage: adding SAG, SHH and CHIR99021;
Third stage: adding SHH, SAG and FGF8b;
Fourth stage: adding SHH and FGF8b.

3. The method according to claim 1, wherein, said multiple stages for induction with supplementary components are:
First stage: adding 1~15µM DMH-1, 200~1000ng/mL SHH, 0.1~1µM CHIR99021;
Second stage: adding 0.1~5µM SAG, 50~300ng/ml SHH, 0.1~1µM CHIR99021;
Third stage: adding 5~100 ng/ml SHH, 0.1~5µM SAG, 5~200 ng/ml FGF8b;
Fourth stage: adding 5∼100 ng/ml SHH, 5~80 ng/ml FGF8b.

4. The method according to claim 3, wherein, said multiple stages for induction with supplementary components are:
First stage: adding 10±5 µM SB431542, 2±1 µM DMH-1, 500±200 ng/ml SHH, 0.4±0.2µM CHIR99021;
Second stage: adding 2±1µM SAG, 100±50 ng/ml SHH, 0.4±0.2µM CHIR99021;
Third stage: adding 20±10ng/ml SHH, 0.5±0.2µM SAG, 100±50 ng/ml FGF8b;
Fourth stage: adding 20±10 ng/ml SHH, 20±10 ng/ml FGF8b.

5. The method according to claim 1 or 2, wherein, in said multiple stages:
First stage: culturing for 6~8 days from the beginning; preferably 7±0.5 days;
Second stage: culturing for 6∼8 days to 11∼ 13 days; preferably 12±0.5 days;
Third stage: culturing for 11∼13 days to 18~20 days; preferably 19±0.5 days;
Fourth stage: culturing for 18~20 days to 31~33 days; preferably 32±0.5 days.

6. The method according to claim 1, wherein the stem cells comprise: embryonic stem cells or induced pluripotent stem cells; preferably, the stem cells are human stem cells, the embryonic stem cells or induced pluripotent stem cells are human embryonic stem cells or human induced pluripotent stem cells.

7. A midbrain substantia nigra dopaminergic nerve cell, wherein it is prepared by the method of any one of claims 1 to 6.

8. A midbrain substantia nigra dopaminergic nerve cell, wherein it expresses molecular markers of midbrain substantia nigra dopaminergic neurons after differentiation for 5∼10 days, said molecular markers comprising tyrosine hydroxylase, FOXA2, EN1, LMX1A, NURR1 and/or GIRK2; while it rarely expresses the marker CALB of the ventral tegmental area dopaminergic neurons.

9. The midbrain substantia nigra dopaminergic nerve cell according to claim 7 or 8, wherein, after the midbrain substantia nigra dopaminergic nerve cell being transplanted into the substantia nigra of brain and differentiation, A9 mDA neurons are obtained the axons thereof can specifically project to the target brain area-dorsal striatum which is innervated by endogenous substantia nigra dopaminergic neurons; and/ or
the obtained A9 mDA neurons themselves exhibit the classic electrophysiological characteristics of the endogenous substantia nigra dopaminergic neurons, comprising a low-frequency spontaneous discharge frequency, and can induce sag by means of hyperpolarizing current stimulation; and/ or
the obtained A9 mDA neurons in the substantia nigra or striatum of brains can alleviate motor deficits.

10. The midbrain substantia nigra dopaminergic nerve cell according to claim 7 or 8, wherein, the midbrain substantia nigra dopaminergic nerve cell is A9 mDA nerve cell; preferably, more than 80% of the cells express A9 mDA marker GIRK2 after differentiation for 5~10 days; more preferably, more than 85% of the cells express A9 mDA marker GIRK2.

11. The midbrain substantia nigra dopaminergic nerve cell according to claim 7 or 8, wherein, after differentiation for 5~10 days, more than 40% of total cells or more than 50% of the TUJ1+ neurons exhibit the characteristics of molecular markers; more preferably, more than 50% of total cells or more than 60% of the TUJ1+ neurons exhibit the characteristics of molecular markers.

12. A midbrain substantia nigra dopaminergic neuron obtained from the differentiation of the midbrain substantia nigra dopaminergic nerve cell of any of claims 7 to 11; preferably, it expresses molecular markers of midbrain substantia nigra dopaminergic neurons, comprising TH, FOXA2, EN1, LMX1A, NURR1 and/or or GIRK2, while it rarely expresses the marker CALB of the ventral tegmental area dopaminergic neurons.

13. A use of the midbrain substantia nigra dopaminergic nerve cell according to any of claims 7 to 11 for preparing preparations in treating neurodegenerative diseases.

14. A preparation for treating neurodegenerative diseases, wherein, it comprises: the midbrain substantia nigra dopaminergic nerve cell according to any of claims 7 to 11; and pharmaceutically acceptable carriers.

15. The use according to claim 14, wherein, the preparation is also used:
as a graft for transplantation into the substantia nigra or striatum of the brain;
for preventing or treating motor deficits.

16. The use according to claim 15, wherein, the neurodegenerative diseases comprise: Parkinson's disease, Alzheimer's disease, Lewy body dementia, Huntington's disease, amyotrophic lateral sclerosis, nerve damage.

17. A method for screening substances for improving neurodegenerative diseases, wherein the method comprises:
(1) Treating a model system by a candidate substance, wherein the model system is neural circuit damaged or neural function damaged which comprises midbrain dopaminergic neurons; and
(2) Evaluating, if the candidate substance can statistically promote dopaminergic neurons to repair damaged neural circuits in the brain or promote their remodeling of neural functions, then the candidate substance is a useful substance for repairing damaged neural circuits or reconstructing neural functions.

18. The method according to claim 17, wherein, the model system in step (1) is an animal model system, a tissue model system, an organ model system, a cell model system.

19. The method according to claim 17, wherein, step (2) comprises:
observing the influence of the candidate substance to midbrain dopaminergic neurons, if it promotes midbrain dopaminergic neurons to repair nigra-striatal pathway, then it is a useful substance for repairing damaged neural circuits or reconstructing neural functions; or
observing the influence of the candidate substance to midbrain dopaminergic neurons, if it promotes pre- and post-synaptic integration of midbrain dopaminergic neurons, then it is a useful substance for repairing damaged neural circuits or reconstructing neural functions; or
observing the influence of the candidate substance to midbrain dopaminergic neurons, if it promotes the projection of axons of midbrain dopaminergic neurons to the dorsal region of striatum, then it is a useful substance for repairing damaged neural circuits or reconstructing neural functions; or
observing the influence of the candidate substance to midbrain dopaminergic neurons, if it promotes the neural fiber formation of midbrain dopaminergic neurons, along with the endogenous nigra-striatal neural connected pathway, specific growth and extension to its endogenous target area-striatum to form neural connections with striatal neurons, and projection to the striatum, then it is a useful substance for repairing damaged neural circuits or reconstructing neural functions.

20. The method according to claim 17, wherein, the model system is an animal system, the animal has motor deficits, wherein the method also comprises: evaluating the motor abilities of animals, if the candidate substance can alleviate motor deficits, then it is a useful substance for repairing damaged neural circuits or reconstructing neural functions.
